# EUROPEAN PATENT APPLICATION

(11) **EP 2 053 393 A1**
(43) Date of publication of application: **29.04.2009**
(21) Application number: 07792236.7
(22) Date of filing: 02.08.2007
(51) Int. Cl.: G01N 33/15, A61K 31/365, A61K 31/496, A61K 45/00, A61P 35/00, C07D 407/04, C07D 495/04, G01N 33/50, G01N 33/536

(54) **PLADIENOLIDE TARGET MOLECULE, COMPOUND CAPABLE OF BINDING TO THE TARGET MOLECULE, AND SCREENING METHOD FOR THE COMPOUND**

(30) Priority: 02.08.2006 US 821199 P
(71) Applicant: Eisai R&D Management Co., Ltd., Tokyo 112-8088 (JP)
(72) Inventor: KOTAKE, Yoshihiko, Tsukuba-shi Ibaraki 300-2635 (JP); SAGANE, Koji, Tsukuba-shi Ibaraki 300-2635 (JP); OWA, Takashi, Tsukuba-shi Ibaraki 300-2635 (JP); MIZUI, Yoshiharu, Tsukuba-shi Ibaraki 300-2635 (JP); SHIMIZU, Hajime, Tsukuba-shi Ibaraki 300-2635 (JP); KIYOSUE, Yuko, Kobe-shi Hyogo 650-0047 (JP)
(74) Representative: Woods, Geoffrey Corlett
(86) International application number: PCT/JP2007/065574
(87) International publication number: WO 2008/016187

(57) **Abstract**

A method of measuring the binding activity of a test compound to a splicing factor 3b (SF3b), which comprises the following steps of:
(a) contacting a labeled pladienolide compound and a test compound with a cell or a cell fraction; and
(b) measuring the distribution of the bound labeled compound. The method enables to screen for a novel active compound capable of acting on (binding to) a pladienolide target molecule or the like.

## Description

### FIELD OF THE INVENTION

The present invention relates to target molecules of pladienolides and the derivatives thereof, compounds binding to the target molecules, and a screening method thereof.

### BACKGROUND ART

As a result of studies regarding anticancer agents conducted in recent years, novel kinase inhibitors have been successively found. Such kinase inhibitors contribute to the treatment of cancer patients or the improvement of QOL. However, cancer treatment results obtained with the use of anticancer agents have not yet been sufficient, and thus it is strongly desired that a novel anticancer agent be developed. In particular, the development of a novel anticancer agent based on a new drug-discovery target is expected, not only to treat cancer patients who cannot obtain sufficient therapeutic effects from the existing agents, but also to construct a new treatment strategy when it is used together with such existing agents.

It has been known that pladienolides exhibit excellent antitumor activity in *vitro* and *in vivo* (International Publications WO02/060890, WO03/099813, WO04/011661, and WO04/011459). It has been shown that pladienolides have anticancer spectra that completely differ from those of the existing antitumor agents, and suggested that they have novel action mechanisms. However, detailed action mechanisms such as *in vivo* target molecules have not yet been clarified.

### DISCLOSURE OF THE INVENTION

Isolation and identification of target molecules to which pladienolides bind result in the finding of a new drug-discovery target. Thus, the present inventors have designed and synthesized various probe compounds based on pladienolides, and they have made an attempt to identify their target molecules.

Objects of the present invention are to identify target molecules that exhibit the physiological activity of pladienolides, to provide probe compounds that are used in the above identification, and to provide a method of screening novel active compounds that act on (bind to) the target molecules of pladienolides, using newly identified target molecule compounds and/or probe-related compounds.

In the aforementioned background, the present inventors have conducted intensive studies directed towards identifying target molecules to which pladienolides specifically bind *in vivo.* As a result, the inventors have found that pladienolides bind to a factor known as SAP130 (which is also referred to as splicing factor 3b3 or SF3b3), so as to modify the functions thereof and the functions of a complex in which the SAP130 is a constituent, thereby exhibiting the physiological activity. Herein, SAP130 is a component of SF3b (splicing factor 3b, Will Cl et. al, EMBO, 2001, 20(16), 4536-46), and SF3b is a constitutional factor of U2 snRNP as a splicing machinery.

A compound that has an influence upon the functions of SF3b is:
(1) likely to have a great influence upon transcription in general based on the close relationship between splicing and transcription (Tom Maniatis & Robin Reed, Nature, 2002, 416, 499-507; Nick J. Proudfoot, Cell, 2002, 108, 501-512);
(2) SF3b is also considered to have an influence upon a cell cycle as it has been suggested that SF3b is associated with a cell cycle via the binding of SF3b to Cyclin E (Mol Cell Biol. 1998 Aug; 18(8): 4526-36)..

From such assumptions, it is considered that SF3b is promising as a novel target of anticancer agents.

Moreover, using newly designed and synthesized probe compounds, novel physiologically active substances that bind to SAP130, SF3b, and U2snRNP can be searched. The inventors have found that antitumor agents that bind to such molecules can be screened, thereby completing the present invention.

That is, the present invention is as follows.
(1) A method of measuring the binding activity of a test compound to a splicing factor 3b (SF3b), which comprises the following steps of:
   (a) contacting a labeled compound represented by the following formula (I) and a test compound with a cell or a cell fraction; and
   (b) measuring the distribution of the bound labeled compound, 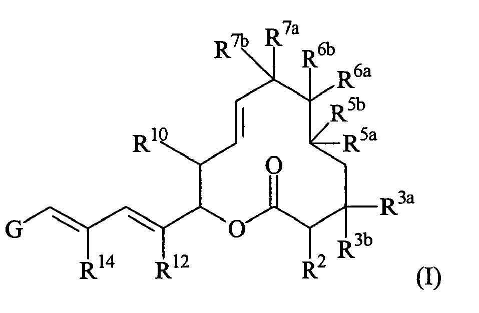 [wherein, in the above formula (I),
      R², R¹⁰, R¹², and R¹⁴ are the same as or different from one another and each represents hydrogen or methyl;
      R^{3a}, R^{3b}, R^{5a}, R^{5b},R^{6a}, and R^{6b} are the same as or different from one another and each represents,
      (1) hydrogen,
      (2) hydroxy,
      (3) any one of the following groups, each of which may have substituent(s),
         (a) C₁₋₂₂ alkyl,
         (b) C₁₋₂₂ alkoxy,
         (c) ArCH₂O- (wherein Ar represents C₆₋₁₄ aryl or 5- to 14-membered ring heteroaryl, each of which may have substituent(s)),
         (d) C₂₋₂₂ acyloxy,
         (e) C₃₋₂₂ unsaturated acyloxy,
         (f) -OCOR^{CO} (wherein R^{CO} represents
            (i) C₆₋₁₄ aryl,
            (ii) 5- to 14-membered ring heteroaryl,
            (iii) C₁₋₂₂ alkoxy,
            (iv) unsaturated C₂₋₂₂ alkoxy,
            (v) C₆₋₁₄ aryloxy, or
            (vi) 5- to 14-membered ring heteroaryloxy, each of which may have substituent(s)),
         (g) C₁₋₂₂ alkylsulfonyloxy,
         (h) benzenesulfonyloxy, or
         (i) -OSiR^{s1}R^{s2}R^{s3} (wherein R^{s1}, R^{s2}, and R^{s3} are the same as or different from one another and each represents methyl, ethyl, i-propyl, t-butyl, or phenyl),
      (4) halogen, or
      (5) -R^{M}-NR^{N1}R^{N2} {wherein R^{M} represents a single bond or -O-CO-; and R^{N1} and R^{N2} are as follows,
         1) R^{N1} and R^{N2} are the same as or different from each other and each represents
            (a) hydrogen or
            (b) any one of the following groups, each of which may have substituent(s):
               (i) C₁₋₂₂ alkyl,
               (ii) unsaturated C₃₋₂₂ alkyl,
               (iii) C₂₋₂₂ acyl,
               (iv) unsaturated C₃₋₂₂ acyl,
               (v) C₆₋₁₄ aryl,
               (vi) 5- to 14-membered ring heteroaryl,
               (vii) benzyl,
               (viii) C₁₋₂₂ alkylsulfonyl or
               (ix) benzenesulfonyl, or
         2) NR^{N1}R^{N2} may together represent 3- to 14-membered ring nitrogen-containing nonaromatic hetero ring, which may have substituent(s)};
R^{7a} and R^{7b} are as follows,
(1) R^{7a} and R^{7b} are different from each other and each independently represents,
   1) hydrogen,
   2) -OR^{H} (wherein R^{H} represents hydrogen, methyl, or acetyl),
   3) -OR^{D} {wherein R^{D} represents any one of the following groups, each of which may have substituent(s),
      (i) C₁₋₂₂ alkyl (in the case of methyl, it must have substituent(s)),
      (ii) -CH₂Ar,
      (iii) C₃₋₂₂ acyl,
      (iv) C₃₋₂₂ unsaturated acyl,
      (v) -COR^{co},
      (vi) C₁₋₂₂ alkylsulfonyl,
      (vii) benzenesulfonyl or
      (viii) -SiR^{s1}R^{s2}R^{s3}}, or
   4) -R^{M}-NR^{N1}R^{N2}, or
(2) R^{7a} and R^{7b} together represents
   1) a ketone structure (=O) or
   2) an oxime structure (=NOR^{OX}, wherein R^{OX} represents
      (a) C₁₋₂₂ alkyl,
      (b) unsaturated C₃₋₂₂ alkyl,
      (c) C₆₋₁₄ aryl,
      (d) 5- to 14-membered ring heteroaryl, or
      (e) benzyl, each of which may have substituent(s));
         further,
         R^{3a} and R^{3b} may together represent a ketone structure (=O) or an oxime structure (=NOR^{OX});
         still further,
         R^{6a} and R^{6b} may together represent a spiro-oxirane ring or exomethylene; and still further,
         either R^{6a} or R^{6b} and either R^{7a} or R^{7b} may together form a 1,3-dioxolane ring;
G represents, 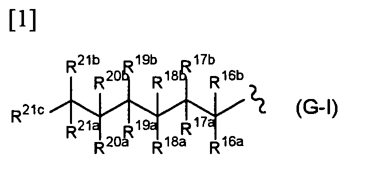 {wherein R^{16a} and R^{16b} are the same as or different from each other and each represents hydrogen, methyl, or hydroxy;
R^{17a}, R^{17b}, R^{18a} R^{18b}, R^{19a} R^{19b}, R^{20a}, R^{20b}, R^{21a}, and R^{21b} are the same as or different from one another and each represents,
(1) hydrogen,
(2) methyl, which may have substituent(s),
(3) -OR^{H},
(4) -OR^{D},
(5) halogen, or
(6) -R^{M}-NR^{N1}R^{N2};
R^{21c} represents
(1) hydrogen or
(2) 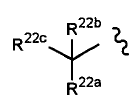 (wherein R^{22a}, R^{22b}, and R^{22c} are the same as or different from one another and each represents
   (a) hydrogen,
   (b) methy
   (c) hydroxy,
   (d) -OR^{H},
   (e) -OR^{D},
   (f) -R^{M}-NR^{N1}R^{N2}, or
   (g) halogen;
   further,
   either R^{18a} or R^{18b} and either R^{19a} or R^{19b} may together form a single bond, so as to represent a partial structure 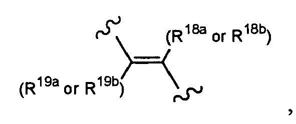 or they may bind to oxygen, so as to represent a partial structure 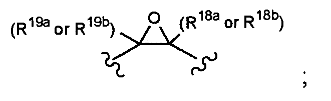 still further,
   either R^{19a} or R^{19b} and either R^{20a} or R^{20b} may together form a single bond, so as to represent 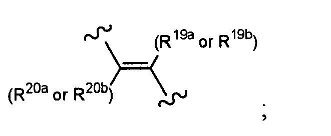 still further,
   R^{21a} and R^{21b} may together represent (a) a ketone structure (=O) or (b) an oxime structure (=NOR^{OX});
   still further,
   either R^{21a} or R^{21b} and either R^{22a} or R^{22b} may together represent a partial structure 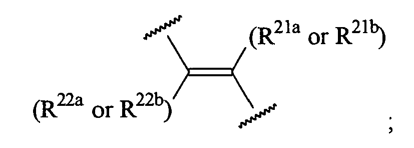 still further,
   either R^{19a} or R^{19b} and either R^{21a} or R^{21b} may together represent a partial structure 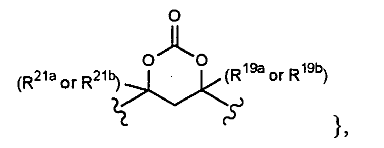 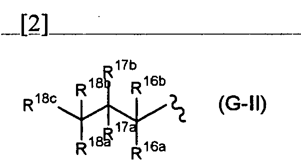 {wherein R^{16a}, R¹⁶⁶, R^{17a}, R^{17b}, R^{18a}, and R^{18b} have the same definitions as those described in formula (G-I); and R^{18c} represents (1) hydrogen or (2) the formula: 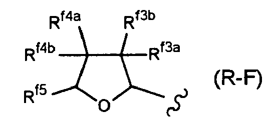 (wherein R^{f3a}, R^{f3b} , R^{f4a}, and R^{f4b} are the same as or different from one another and each represents hydrogen, methyl, hydroxy, methoxy, or acetoxy, and R^{f5} represents methyl or ethyl)} or 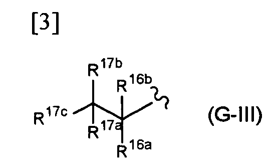 {wherein R^{16a}, R^{16b}, R^{17a}, and R^{17b} have the same definitions as those described in formula (G-I); and R^{17c} represents (1) hydrogen or (2) the formula: 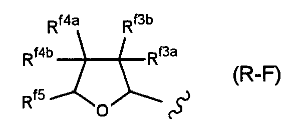 (wherein R^{f3a}, R^{f3b}, R^{f4a}, and R^{f4b} are the same as or different from one another and each represents hydrogen, methyl, hydroxy, methoxy, or acetoxy, and R^{f5} represents methyl or ethyl)}].
(2) The method according to (1) above, wherein the compound represented by the above formula (I) is a compound represented by the following formula (IV): 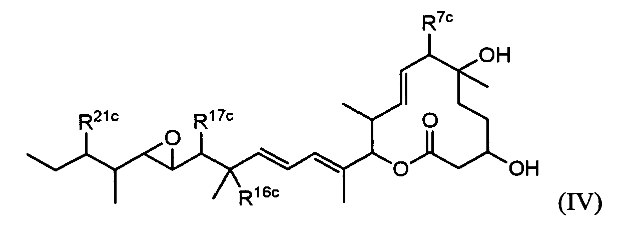 [wherein, in the above formula (IV),
   R^{16c}, R^{17c}, and R^{21c} are the same as or different from one another and each represents hydrogen, hydroxy, or methoxymethyl; and
   R^{7c} represents hydroxy, acetoxy, or -CO-NR^{N1'}R^{N2'} (wherein R^{N1'} and R^{N2}' are the same as or different from each other and each represents hydrogen or C₁₋₆ alkyl)].
(3) The method according to (1) above, wherein the compound represented by the above formula (I) is a compound represented by the following formula (V): 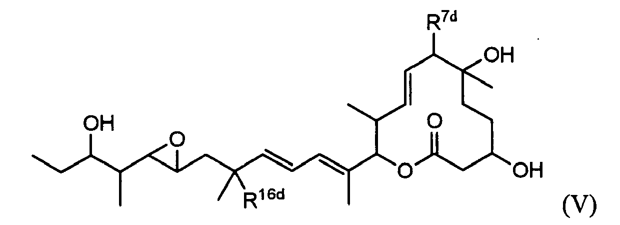 [wherein, in the above formula (V),
   R^{16d} represents hydrogen or hydroxy; and
   R^{7d} represents hydroxy, acetoxy, or O-CO-NHR^{N1}' (wherein R^{N1}' represents C₁₋₆ alkyl)].
(4) The method according to any one of (1) to (3) above, wherein the activity of the test compound to bind to SAP130 in SF3b is measured.
(5) The method according to any one of (1) to (4) above, wherein the label is radioisotope label.
(6) The method according to any one of (1) to (4) above, wherein the label is fluorescent label.
(7) The method according to any one of (1) to (4) above, wherein the label is biotin label.
(8) The method according to any one of (1) to (4) above, wherein the label is a label with a compound that binds to a protein as a result of exposure to light.
(9) The method according to any one of (1) to (4) above, wherein, in the step (b), the amount of the labeled compound that is distributed in nuclei is measured, so as to measure the binding activity of the test compound to SF3b.
(10) The method according to any one of (1) to (4) above, wherein, in the step (b), the amount of the labeled compound that is distributed in nuclear speckles is measured, so as to measure the binding activity of the test compound to SF3b.
(11) The method according to any one of (1) to (4) above, wherein, in the step (b), the pattern of distribution of the labeled compound in nuclear speckles is measured, so as to measure the binding activity of the test compound to SF3b.
(12) The method according to any one of (1) to (9) above, wherein, in the step (b) the amount of the labeled compound that is distributed in SF3b-bound state is measured, so as to measure the binding activity of the test compound to SF3b.
(13) The method according to any one of (1) to (4) above, wherein, in the step (b), the amount of the labeled compound that is distributed in SAP130-bound state is measured, so as to measure the binding activity of the test compound to SF3b.
(14) An anticancer agent binding to SF3b determined to have activity to bind to SF3b by the method according to any one of (1) to (13) above.
(15) An anticancer agent binding to SAP130 determined to have activity to bind to SAP130 by the method according to any one of (1) to (13) above.
(16) A labeled compound that is any of those represented by the following formula (II):

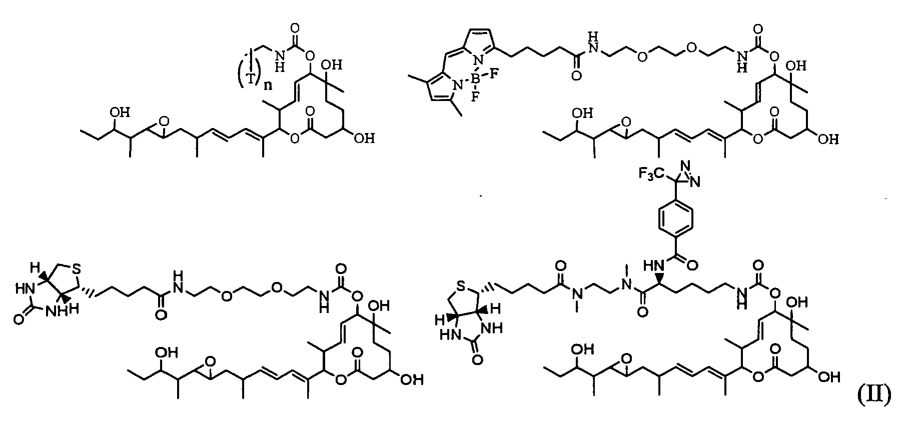

According to the present invention, a novel compound that acts on (binds to) the target molecules of pladienolides can be screened.

### BRIEF DESCRIPTION OF THE DRAWINGS

Figure 1a is a view showing a ¹H-NMR spectrum.
Figure 1b is a view showing ESI-MS.
Figure 1c is a view showing the intracellular distribution of a tritium probe compound. The longitudinal axis of the graph indicates the value of radioactivity in the sample, which has been converted from the value of specific activity of the probe to the number of moles.
Figure 1d is a view showing the intracellular distribution of a tritium probe compound (charcoal assay).
Figure 2 is a view showing the results of a competition assay of pladieno analogues and pladieno derivatives with tritium probes (the correlation between the values of biological activity of competitors and competitive rates).
Figure 3a is a view showing the observation results of intracellular localization of a fluorescent probe.
Figure 3b is a view showing the observation results of intracellular localization of a fluorescent probe (the results of a competition assay of pladienolide compounds).
Figure 4 is a view showing the experimental results of immunoprecipitation of a nuclear fraction prepared from cells treated with a tritium probe.
Figure 5a is a view showing the results of detection of bound proteins by photoaffinity biotin probe treatment.
Figure 5b is a view showing the results of detection of bound proteins by photoaffinity biotin probe treatment (the comparative results of the band positions of SAP155, SAP145, and SAP130).
Figure 6a is a view showing the experimental results of detection of a band shift using GFP-fused SAP145.
Figure 6b is a view showing the experimental results of detection of a band shift using GFP-fused SAP130.
Figure 7 is a view showing the experimental results of a competition assay of pladieno compounds with photoaffinity biotin probes.
Figure 8a is a view showing a representative example of fluorescent probe used in imaging analysis.
Figure 8b is a view showing the results of imaging analysis.

### BEST MODE FOR CARRYING OUT THE INVENTION

The present invention will be described in detail below. The following embodiments are provider for illustrative purposes only, and are not intended to limit the scope of the present invention to such embodiments. The present invention can be carried out in various embodiments without departing from the spirit of the invention.

All publications, patent application publications, patents and other patent documents cited herein are incorporated herein by reference in their entirety. The present specification also incorporates the disclosures of US provisional application 60/821,199 based on which the present application claims priority.

In the present invention, the compound represented by the aforementioned formula (I) is related to a compound group, which is described in International Publications WO02/060890, WO03/099813, WO04/011661, and WO04/011459, and whose antitumor activity has been recognized. The above compound can be used as a compound to be labeled in the present invention. The above compound is referred to as "pladienolides" in the present specification, and it is also referred to as a "pladienolide analogue or derivative" at times. The compound represented by the following formula (IV) or (V) is a preferred example of such pladienolides. 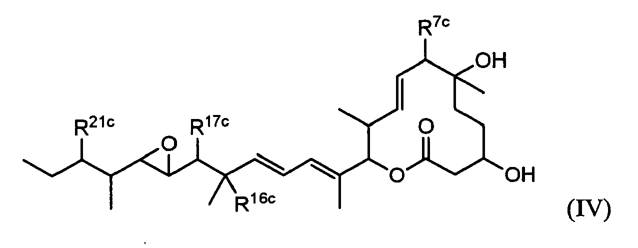 [wherein R^{16c}, R^{17c} and R^{21c} are the same as or different from one another and each represents hydrogen, hydroxy or methoxymethyl;
R^{7c} represents hydroxy, acetoxy, O-CO-NR^{N1'}R^{N2'} (wherein R^{N1'} and R^{N2'} are the same as or different from each other and each represents hydrogen or C₁₋₆ alkyl)] 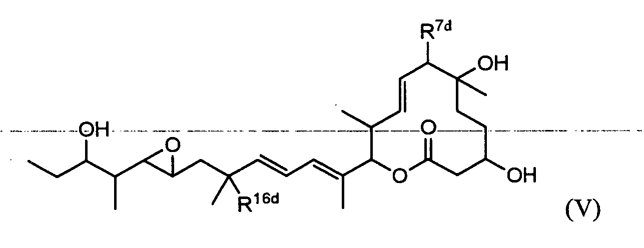 [wherein R¹⁶d represents hydrogen or hydroxy, preferably hydrogen; R^{7a} represents hydroxy, acetoxy, O-CO-NHR^{N1'} (wherein R^{N1'} represents C₁₋₆ alkyl)]

The position to be labeled is not limited, as long as antitumor activity is not lost due to such labeling. It is preferable that the acetyl group at position 7 be modified (labeled). Examples of a labeling method include labeling with a radioisotope and labeling with a fluorochrome (fluorophore), but examples are not limited thereto. In addition, a method of allowing biotin to bind to a molecule to be labeled, followed by detection using avidin that specifically binds to biotin, can also be adopted. However, such a specific bind is not limited to in the case of detection with the combined use of biotin with avidin.

A structure activated by light irradiation to form a covalent bond together with surrounding functional groups (a photoaffinity moiety) may be introduced into a molecule to be labeled, enabling to prevent the labeled molecule from dissociation in a treatment with a protein denaturant, for example, by SDS, after a covalent bond was formed by light irradiation.

A method of synthesizing a labeled compound is specifically described in Examples A1 to A5.

In the present specification, the term "labeled compound" is used to mean a compound obtained by labeling the compound represented by the formula (I), and a preferred example of such a labeled compound is any compound represented by the formula (II).

An example of the screening method of examining whether or not a test compound acts on (binds to) U2 snRNP, preferably SF3b, and more preferably SAP130 is a method comprising (a) contacting the labeled compound represented by the following formula (I) and a test compound with cells or a cell fraction, and (b) measuring distribution of the bound labeled compound. More specific examples are the following methods. It is to be noted that the term "contact" is used to mean that the labeled compound represented by the formula (I) and the test compound are allowed to exist with the cells or the cell fraction in a single reaction system or culture system. For example, a case where the labeled compound represented by the formula (I) and the test compound are added to a cell culture vessel, a case where the cells are cultured in the presence of the labeled compound represented by the formula (I) and the test compound, and a case where the labeled compound represented by the formula (I) and the test compound are mixed with a solution of the cell fraction, and the like are included.

### 1. Examination method utilizing activity of test compound to suppress distribution of labeled pladienolides into nuclei

(1) After cells have been cultured in the presence of a test compound and labeled pladienolides, the cells are fractionated into a nuclear fraction and other fractions. When the quantities of the labeled pladienolides in the nuclear fraction in the presence of the test compound become smaller than those in the absence of the test compound, for example, when the aforementioned quantities are 50% or less, preferably 70% or less, and more preferably 90% or less, it can be determined that the test compound has the activity of acting on (binding to) U2 snRNP, preferably SF3b, and more preferably SAP130.
   In the present invention, after the cells have been fractionated into a nuclear fraction and other fractions, it is also possible that the test compound and the labeled pladienolides be added to each fraction, followed by incubation for a suitable period of time, and that the quantities of the labeled pladienolides in the nuclear fraction be then measured. In addition, the test compound and the labeled pladienolides may be simultaneously added to each fraction, or either the test compound or the labeled pladienolides may be previously added thereto. It is preferable that the test compound be added in advance.
   As described in Example B2, there is a strong correlation between the antitumor activity of the test compound and the activity of the test compound to suppress distribution of the labeled pladienolides into a nuclear fraction. Accordingly, the method of the present invention makes it possible to screen a compound, which acts on (binds to) U2 snRNP, preferably SF3b, and more preferably SAP130, so as to exhibit antitumor activity.
(2) Distribution of the labeled pladienolides into nuclei can be examined by optically analyzing cells (for example, quantification of the labeled pladienolides distributed into the nuclei, on the basis of the image thereof, using a microscope). Thereafter, the result obtained by such optical analysis is used as an indicator, and a compound that suppresses distribution of the labeled pladienolides into the nuclei can be examined. An example of the method of examining distribution of the labeled pladienolides into the nuclei by image analysis will be described in Example B9.

### 2. Examination method utilizing activity of test compound to suppress distribution of labeled pladienolides into nuclear speckles

Cells are cultured in the presence of a test compound and labeled pladienolides. The cells can be optically analyzed (for example, quantification of the labeled pladienolides distributed into nuclear speckles, on the basis of the image thereof, using a microscope), so as to examine distribution of the labeled pladienolides into the nuclear speckles. Thereafter, the result obtained by such optical analysis is used as an indicator, and a compound that suppresses distribution of the labeled pladienolides into the nuclear speckles can be examined.

Such nuclear speckles can be stained with an antibody reacting with a protein existing in the nuclear speckles, such as an anti-SC-35 antibody. As with the stained nuclear speckles, the labeled pladienolides distributed therein can be quantified, so as to measure distribution of the labeled pladienolides into the nuclear speckles.

When the quantities of the labeled pladienolides in the nuclear speckles in the presence of the test compound become smaller than those in the absence of the test compound, for example, when the aforementioned quantities are 50% or less, preferably 70% or less, and more preferably 90% or less, it can be determined that the test compound has the activity of acting on (binding to) U2 snRNP, preferably SF3b, and more preferably SAP130.

In addition, such cells are subjected to optical observation or image analysis, and based on the pattern of distribution of the labeled compound in the nuclear speckles, the binding activity of the test compound to SF3b can be measured. For example, when such nuclear speckles agglutinate and they are observed as enlarged nuclear speckles, it can be determined that the test compound has the activity of acting on (binding to) U2 snRNP, preferably SF3b, and more preferably SAP130.

As described in Example B3, there is a correlation between the antitumor activity of the test compound and the activity of the test compound to suppress distribution of the labeled pladienolides into nuclear speckles. Accordingly, the method of the present invention makes it possible to screen a compound, which acts on (binds to) U2 snRNP, preferably SF3b, and more preferably SAP130, so as to exhibit antitumor activity.

### 3. Examination method utilizing activity of test compound to suppress binding of labeled pladienolides to U2snRNP, SF3b or SAP130

Cells are cultured in the presence of a test compound and labeled pladienolides, preferably photoaffinity labeled pladienolides. When such photoaffinity labeled pladienolides are used as labeled pladienolides, light is applied to the culture. After applying light, the cells are solubilized. Thereafter, proteins contained in the solubilized components are fractionated, preferably fractionated by SDS-PAGE, and the labeled pladienolides in a fraction that contains SAP130 are then quantified.

Preferably, light can be applied to an immunoprecipitation sample obtained by treating solubilized components obtained by solubilizing cells cultured in the presence of a test compound and photoaffinity labeled pladienolides with an anti-SAP155 antibody, an anti-SAP145 antibody, an anti-SAP120 antibody, an anti-U2B" antibody or the like, and preferably with an anti-SAP155 antibody. Moreover, it is also possible that an immunoprecipitation sample obtained by treating solubilized components with an anti-SAP155 antibody, an anti-SAP145 antibody, an anti-SAP120 antibody, an anti-U2B" antibody or the like, and preferably with an anti-SAP155 antibody, be treated with a test compound and photoaffinity labeled pladienolides, and that light be then applied to the resultant immunoprecipitation sample.

The wavelength used in light irradiation is not particularly limited. A wavelength that activates a used photoaffinity probe is preferable.

When the quantities of the labeled pladienolides in a U2 snRNP, SF3b, or SAP130 fraction in the presence of a test compound become smaller than those in the absence of the test compound, for example, when the aforementioned quantities are 50% or less, preferably 70% or less, and more preferably 90% or less, it can be determined that the test compound has the activity of acting on (binding to) U2 snRNP, preferably SF3b, and more preferably SAP130.

In the present invention, it is also possible that the test compound and the labeled pladienolides be added to the solubilized components after solubilizing the cells, followed by incubation for a suitable period of time, and that the quantities of the labeled pladienolides contained in the SAP130 fraction be then measured. In addition, the test compound and the labeled pladienolides may be simultaneously added to the solubilized components, or either the test compound or the labeled pladienolides may be previously added thereto. It is preferable that the test compound be added in advance.

SAP130 can be fused to a protein acting as a tag, such as GFP, so as to form a fused protein, and such a fused protein is then allowed to be expressed in certain cells. The use of such cells enables easy fractionation of SAP130, and thus it is preferable. Moreover, an immunoprecipitation experiment using an anti-GFP antibody is carried out on cells wherein SAP 145 has been allowed to be expressed in the form of a fusion protein with a protein acting as a tag, such as GFP, so that SF3b can be fractionated.

As described in Example B7, there is a correlation between the antitumor activity of the test compound and the activity of the test compound to suppress distribution of the labeled pladienolides into the SAP130 fraction. Accordingly, the method of the present invention makes it possible to screen a compound, which acts on (binds to) SAP130 so as to exhibit antitumor activity.

A compound discovered by the present screening system has an effect on RNA splicing, and thus it is useful as an anticancer agent. Further, it is considered that this compound is also useful as a therapeutic agent for other diseases that are considered to be developed due to abnormal splicing, such as neurodegenerative diseases (e.g. familial Alzheimer's disease), dementia (e.g. frontotemporal dementia (Hutton, M. et al., Nature, 393:702-705, 1998)), mental disorders (e.g. familial dysautonomia (Hims MM et al., J Mol Med. 2007, 85(2):149-61. Epub 2007)), amyotrophic and myotonic degenerative diseases (e.g. spinal muscular atrophy and myotonic dystrophy), progeria, brain tumor, familial hypercholesterolemia, familial isolated growth hormone deficiency type II (Faustino, N.A. et al., Genes Dev.,17:419-437, 2006), and autoimmune disease. Moreover, it is also considered that the compound is further useful as a therapeutic agent for infectious diseases involving viruses that utilize splicing during their growth process, such as retrovirus (in particular HIV).

The compound of formula (I) is described below.

In the formula (I), R², R¹⁰, R¹², and R¹⁴ are the same as or different from one another and each represents hydrogen or methyl.

In the formula (I), R^{3a}, R^{3b}, R^{5a}, R^{5b}, R^{6a}, and R^{6b} are the same as or different from one another and each represents any one of (1) to (5) below:
(1) hydrogen,
(2) hydroxy,
(3) any one of the following groups, each of which may have substituent(s),
   (a) C₁₋₂₂ alkyl,
   (b) C₁₋₂₂ alkoxy,
   (c) ArCH₂O- (wherein Ar represents C₆₋₁₄ aryl or 5- to 14-membered ring heteroaryl, each of which may have substituent(s)),
   (d) C₂₋₂₂ acyloxy,
   (e) C₃₋₂₂ unsaturated acyloxy,
   (f) -OCOR^{CO} (wherein R^{CO} represents
      (i) C₆₋₁₄aryl,
      (ii) 5- to 14-membered ring heteroaryl,
      (iii) C₁₋₂₂ alkoxy,
      (iv) unsaturated C₂₋₂₂ alkoxy,
      (v) C₆₋₁₄aryloxy, or
      (vi) 5- to 14-membered ring heteroaryloxy, each of which may have substituent(s)),
   (g) C₁₋₂₂ alkylsulfonyloxy
   (h) benzenesulfonyloxy, or
   (i) -OSiR^{s1}R^{s2}R^{s3} (wherein R^{s1}, R^{s2}, and R^{s3} are the same as or different from one another and each represents methyl, ethyl, i-propyl, t-butyl, or phenyl),
(4) halogen, or
(5) -R^{M}-NR^{N1}R^{N2} {wherein R^{M} represents a single bond or -O-CO-.

In this case, R^{N1} and R^{N2} represent 1) or 2) as follows:
1) R^{N1} and R^{N2} are the same as or different from each other and each represents
   (a) hydrogen or
   (b) any one of the following groups, each of which may have substituent(s):
      (i) C₁₋₂₂ alkyl,
      (ii) unsaturated C₃₋₂₂ alkyl,
      (iii) C₂₋₂₂ acyl,
      (iv) unsaturated C₃₋₂₂ acyl,
      (v) C₆₋₁₄ aryl,
      (vi) 5- to 14-membered ring heteroaryl,
      (vii) benzyl,
      (viii) C₁₋₂₂ alkylsulfonyl or
      (ix) benzenesulfonyl, or
2) NR^{N1}R^{N2} may together represent 3- to 14-membered ring nitrogen-containing nonaromatic hetero ring, which may have substituent(s).

In the formula (I), R^{7a} and R^{7b} represent (1) or (2) below:
(1) R^{7a} and R^{7b} are different from each other and each independently represents,
   1) hydrogen,
   2) -OR^{H} (wherein R^{H} represents hydrogen, methyl, or acetyl),
   3) -OR^{D} {wherein R^{D} represents any one of the following groups, each of which may have substituent(s),
      (i) C₁₋₂₂ alkyl (in the case of methyl, it must have substituent(s)),
      (ii) -CH2Ar,
      (iii) C₃₋₂₂ acyl,
      (iv) C₃₋₂₂ unsaturated acyl,
      (v) -COR^{CO},
      (vi) C₁₋₂₂ alkylsulfonyl,
      (vii) benzenesulfonyl or (viii) -SiR^{s1}R^{s2}R^{s3}}, or
   4) -R^{M}-NR^{N1}R^{N2}, or
(2) R^{7a} and R^{7b} together represents
   1) a ketone structure (=O) or
   2) an oxime structure (=NOR^{OX}, wherein R^{OX} represents
      (a) C₁₋₂₂ alkyl,
      (b) unsaturated C₃₋₂₂ alkyl,
      (c) C₆₋₁₄ aryl,
      (d) 5- to 14-membered ring heteroaryl, or
      (e) benzyl, each of which may have substituent(s)).

In the formula (I), R^{3a} and R^{3b} may together represent a ketone structure (=O) or an oxime structure (=NOR^{OX}). Further, R^{6a} and R^{6b} may together represent a spiro-oxirane ring or exomethylene. Further, either R^{6a} or R^{6b} and either R^{7a} or R^{7b} may together form a 1,3-dioxolane ring.

In the formula (I), G represents any one of [1] to [3] below.

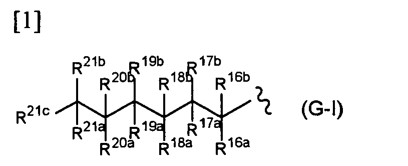

In the formula (G-I), R^{16a} and R^{16b} are the same as or different from each other and each represents hydrogen, methyl, or hydroxy.
In the formula (G-I), R^{17a}, R^{17b}, R^{18a}, R^{18b}, R^{19a}, R^{19b}, R^{20a}, R^{20b}, R^{21a}, and R^{21b} are the same as or different from one another and each represents any one of (1) to (6) below:
(1) hydrogen,
(2) methyl, which may have a substituent,
(3) -OR^{H},
(4) -OR^{D},
(5) halogen, or
(6) -R^{M}-NR^{N1}R^{N2}.

In the formula (G-I), R^{21c} represents any one of (1) and (2) below:
(1) hydrogen or
(2) 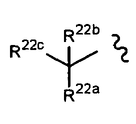 (wherein R^{22a}, R^{22b}, and R^{22c} are the same as or different from one another and each represents
   (a) hydrogen,
   (b) methyl,
   (c) hydroxy,
   (d) -OR^{H},
   (e) -OR^{D},
   (f) -R^{M}-NR^{N1}R^{N2}, or
   (g) halogen.
In the formula (G-I), further, either R^{18a} or R^{18b} and either R^{19a} or R^{19b} may together form a single bond, so as to represent a partial structure 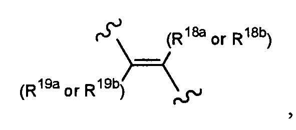 or they may bind to oxygen, so as to represent a partial structure 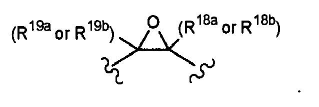 In the formula (G-I), still further, either R^{19a} or R^{19b} and either R^{20a} or R^{20b} may together form a single bond, so as to represent 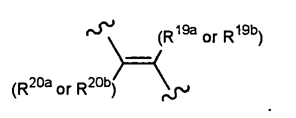 In the formula (G-I), still further, R^{21a} and R^{21b} may together represent (a) a ketone structure (=O) or (b) an oxime structure (=NOR^{OX}).
In the formula (G-I), still further, either R^{21a} or R^{21b} and either R^{22a} or R^{22b} may together represent a partial structure 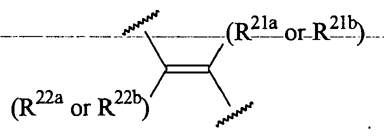 In the formula (G-I), still further, either R^{19a} or R^{19b} and either R^{21a} or R^{21b} may together represent a partial structure

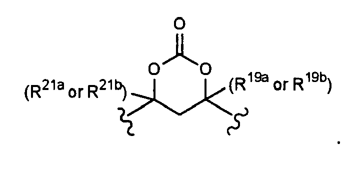

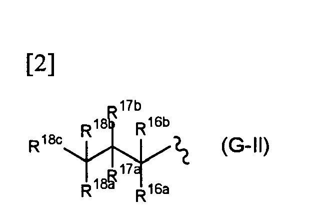

In the formula (G-II), R^{16a}, R^{16b}, R17^{a}, R^{17b}, R^{18a}, and R^{18b} have the same definitions as those described in formula (G-I).
In the formula (G-II), R^{18c} represents (1) or (2) below:
(1) hydrogen or
(2) the formula: 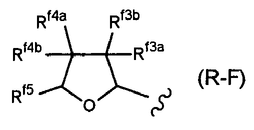 (wherein R^{f3a}, R^{f3b}, R^{f4a}, and R^{f4b} are the same as or different from one another and each represents hydrogen, methyl, hydroxy, methoxy, or acetoxy, and R^{f5} represents methyl or ethyl).

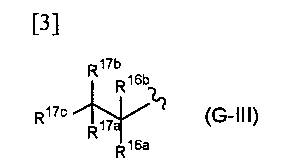

In the formula (G-III), R^{16a}, R^{16b}, R^{17a}, and R^{17b} have the same definitions as those described in formula (G-I). In the formula (G-III), R^{17c} represents (1) or (2) below:
(1) hydrogen or
(2) the formula: 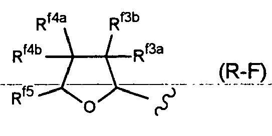 (wherein R^{f3a}, R^{f3b}, R^{f4a}, and R^{f4b} are the same as or different from one another and each represents hydrogen, methyl, hydroxy, methoxy, or acetoxy, and R^{f5} represents methyl or ethyl).

The term "C₁₋₂₂ alkyl" used in the present specification means a linear or branched alkyl group or a cycloalkyl group having 1 to 22 carbon atoms, such as methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, n-hexyl group, 1-ethyl-2-methypropyl group, 1,1,2-trimethylpropyl group, 1-propylpropyl group, 1-methylbutyl group, 2-methylbutyl group, 1,1-dimethylbutyl group, 1,2-dimethylbutyl group, 2,2-dimethylbutyl group, 1,3-dimethylbuty group, 2,3-dimethylbutyl group, 2-ethylbutyl group, 2-methylpentyl group, 3-methylpentyl group, n-heptyl group, n-octyl group, n-nonyl group, n-decyl group, cyclopropyl group, cyclobutyl group, cyclopentyl group, or cyclohexyl group; preferably a linear or branched alkyl group or a cycloalkyl group having 1 to 6 carbon atoms, such as methyl group, ethyl group, n-propyl group, iso-propyl group, n-butyl group, iso-butyl group, sec-butyl group, tert-butyl group, or n-pentyl group; and more preferably, for example, methyl group, ethyl group, propyl group, iso-propyl group, n-butyl group, iso-butyl group, tert-butyl group, cyclopropyl group, cyclobutyl group, cyclopentyl group, or cyclohexyl group.

The term "unsaturated C₃₋₂₂ alkyl" used in the present specification means a linear or branched alkenyl group having 3 to 22 carbon atoms, or a linear or branched alkynyl group having 3 to 22 carbon atoms, such as allyl group, 1-propenyl group, isopropenyl group, 2-methyl-1-propenyl group, 2-methyl-2-propenyl group, 1-butenyl group, 2-butenyl group, 3-butenyl group, 1-pentenyl group, 1-hexenyl group, 1,3-hexanedienyl group, 1,5-hexanedienyl group, 1-propynyl group, 2-propynyl group, 1-butynyl group, 2-butynyl group, 3-butynyl group, 1-ethynyl-2-propynyl group, 2-methyl-3-propynyl group, 1-pentynyl group, 1-hexynyl group, 1-3-hexanediynyl group, or 1,5-hexanediynyl group; preferably a linear or branched alkenyl group having 3 to 10 carbon atoms, or a linear or branched alkynyl group having 3 to 10 carbon atoms, such as allyl group, 1-propenyl group, 2-propenyl group, isopropenyl group, 3-methyl-2-butenyl group, 3,7-dimethyl-2,6-octadienyl group, 1-propynyl group, 2-propynyl group, 1-butynyl group, 2-butynyl group, 3-butynyl group, or 3-methyl-1-propynyl group.

The term "C₁₋₂₂ alkoxy" used in the present specification means a group formed by binding an oxygen atom to the terminus of the above-defined "C₁₋₂₂ alkyl." Examples of a suitable group include methoxy group, ethoxy group, n-propoxy group, iso-propoxy group, n-butoxy group, iso-butoxy group, sec-butoxy group, tert-butoxy group, n-pentyloxy group, iso-pentyloxy group, sec-pentyloxy group, n-hexoxy group, iso-hexoxy group, 1,1-dimethylpropyloxy group, 1,2-dimethylpropoxy group, 2,2-dimethylpropyloxy group, 1-methyl-2-ethylpropoxy group, 1-ethyl-2-methylpropoxy group, 1,1,2-trimethylpropoxy group, 1,2,2-trimethylpropoxy group, 1,1-dimethylbutoxy group, 1,2-dimethylbutoxy group, 2,2-dimethylbutoxy group, 2,3-dimethylbutyloxy group, 1,3-dimethylbutoxy group, 2-ethylbutoxy group, 2-methylpentoxy group, 3-methylpentoxy group, and hexyloxy group; preferably include, for example, methoxy group, ethoxy group, n-propoxy group, iso-propoxy group, iso-butoxy group, and 2,2-dimethylpropyloxy group.

The term "unsaturated C₂₋₂₂ alkoxy" used in the present specification means a group formed by binding an oxygen atom to the terminus of the above-defined "unsaturated C₃₋₂₂ alkyl," vinyl, and ethyl. Examples of a suitable group include vinyloxy group, allyloxy group, 1-propenyloxy group, 2-propenyloxy group, isopropenyloxy group, 2-methyl-1-propenyloxy group, 2-methyl-2-propenyloxy group, 1-butenyloxy group, 2-butenyloxy group, 3-butenyloxy group, 1-pentenyloxy group, 1-hexenyloxy group, 1,3-hexanedienyloxy group, 1,5-hexanedienyloxy group, propargyloxy group, and 2-butynyloxy group; preferably include, allyloxy group, propargyloxy group, and 2-butynyloxy group.

The term "C₆₋₁₄ aryl" used in the present specification means an aromatic hydrocarbon cyclic group having 6 to 14 carbon atoms, and it includes a monocyclic group and a condensed ring such as a bicyclic group or a tricyclic group. Examples thereof are phenyl group, indenyl group, 1-naphthyl group, 2-naphthyl group, azulenyl group, heptalenyl group, indacenyl group, acenaphthyl group, fluorenyl group, phenalenyl group, phenanthrenyl group, and anthracenyl group; of which a preferred example is phenyl group, 1-naphthyl group, or 2-naphthyl group.

The term "5- to 14-membered heteroaryl" used in the present specification means a monocyclic, bicyclic or tricyclic, 5- to 14-membered aromatic heterocyclic group, which comprises one or more heteroatoms selected from the group consisting of a nitrogen atom, a sulfur atom and an oxygen atom. Examples of suitable group include a nitrogen-containing aromatic heterocylic group such as pyrrolyl group, pyridyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, purinyl group, indazolyl group, quinolyl group, isoquinolyl group, quinolizyl group, phthalazyl group, naphthyridinyl group, quinoxalyl group, quinazolinyl group, cinnolinyl group, pteridinyl group, imidazotriazinyl group, pyrazinopyridazinyl group, acridinyl group, phenanthridinyl group, carbazolyl group, carbazolinyl group, perimidinyl group, phenanthrolinyl group, phenacinyl group, imidazopyridinyl group, imidazopyrimidinyl group, pyrazolopyridinyl group, or pyrazolopyridinyl group; a sulfur-containing aromatic heterocyclic group such as thienyl group or benzothienyl group; an oxygen-containing aromatic heterocyclic group such as furyl group, pyranyl group, cyclopentapyranyl group, benzofuryl group, or isobenzofuryl group; an aromatic heterocyclic group containing different types of two or more heteroatoms such as thiazolyl group, isothiazolyl group, benzothiazolyl group, benzothiadiazolyl group, phenothiazinyl group, isoxazolyl group, furazanyl group, phenoxazinyl group, oxazolyl group, isoxazoyl group, benzoxazolyl group, oxadiazolyl group, pyrazolooxazolyl group, imidazothiazolyl group, thienofuranyl group, phlopyrrolyl group, or pyridoxadinyl group, of which a preferred example is thienyl group, furyl group, pyridyl group, pyridazinyl group, pyrimidyl group, or pyrazyl group.

The term "C₆₋₁₄ aryloxy" used in the present specification means a group formed by binding an oxygen atom to the terminus of the above-defined "C₆₋₁₄ aryl." Specific examples include phenyloxy group, indenyloxy group, 1-naphthyloxy group, 2-naphthyloxy group, azulenyloxy group, heptalenyloxy group, indacenyloxy group, acenaphthyloxy group, fluorenyloxy group, phenalenyloxy group, phenanthrenyloxy group, and anthracenyloxy group, of which a preferred example is phenyloxy group, 1-naphthyloxy group, or 2-naphthyloxy group.

The term "5- to 14-membered heteroaryloxy" used in the present specification means a group formed by binding an oxygen atom to the terminus of the above-defined "5- to 14-membered heteroaryl." Specific examples include pyrrolyloxy group, pyridyloxy group, pyridazinyloxy group, pyrimidinyloxy group, pyrazinyloxy group, triazolyloxy group, tetrazolyloxy group, benzotriazolyloxy group, pyrazolyloxy group, imidazolyloxy group, benzimidazolyloxy group, indolyloxy group, isoindolyloxy group, indolizinyloxy group, purinyloxy group, indazolyloxy group, quiolyloxy group isoquinolyloxy group, quinolizyloxy group, phthalazyloxy group, naphthyridinyloxy group, quinoxalyloxy group, quinazolinyloxy group, cinnolinyloxy group, pteridinyloxy group, imidazotriazinyloxy group, pyrazinopyridazinyloxy group, acridinyloxy group, phenanthridinyloxy group, carbazolyloxy group, carbazolinyloxy group, perimidinyloxy group, phenanthrolinyloxy group, phenacinyloxy group, imidazopyridinyloxy group, imidazopyrimidinyloxy group, pyrazolopyridinyloxy group, pyrazolopyridinyloxy group, thienyloxy group, benzothienyloxy group, furyloxy group, pyranyloxy group, cyclopentapyranyloxy group, benzofuryloxy group, isobenzofuryloxy group, thiazolyloxy group, isothiazolyloxy group, benzothiazolyloxy group, benzothiadiazolyloxy group, phenothiazinyloxy group, isoxazolyloxy group, furazanyloxy group, phenoxazinyloxy group, oxazolyloxy group, isoxazoyloxy group, benzoxazolyloxy group, oxadiazolyloxy group, pyrazolooxazolyloxy group, imidazothiazolyloxy group, thienofuranyloxy group, phlopyrrolyloxy group, and pyridoxadinyloxy group, of which a preferred example is thienyloxy group, pyridyloxy group, pyrimidyloxy group, or pyrazyloxy group.

The term "C₂₋₂₂ acyl" used in the present specification means an acyl group with 2 to 22 carbon atoms. Examples of suitable groups include linear or branched acyl groups such as acetyl group, propionyl group, butyryl group, iso-butyryl group, valeryl group, iso-valeryl group, pivalyl group, caproyl group, decanoyl group, lauroyl group, myristoyl group, palmitoyl group, stearoyl group, and arachidoyl group.

Moreover, the term "C₂₋₂₂ acyloxy" used in the present specification means a group having a partial structure corresponding to the aforementioned "C₂₋₂₂ acyl."

The term "unsaturated C₃₋₂₂ acyl" used in the present specification means an acyl group with 3 to 22 carbon atoms having double bond(s) or triple bond(s). Preferred unsaturated C₃₋₂₂ acyl groups include linear or branched acyl groups such as acryl group, propiol group, crotonyl group, iso-crotonyl group, oleinol group, and linolenoyl group.

Moreover, the term "unsaturated C₃₋₂₂ acyloxy" used in the present specification means a group having a partial structure corresponding to the aforementioned "unsaturated C₃₋₂₂ acyl."

The term "C₁₋₂₂ alkylsulfonyl" used in the present specification means a group formed by binding the above-defined "C₁₋₂₂ alkyl" to sulfonyl. Specific examples include methylsulfonyl group, ethylsulfonyl group, n-propylsulfonyl group, and iso-propylsulfonyl group, of which a preferred example is methylsulfonyl group.

The term "C₁₋₂₂ alkylsulfonyloxy" used in the present specification means a group formed by binding an oxygen atom to the terminus of the above-defined "C₁₋₂₂ alkylsulfonyl," such as methylsulfonyloxy group, ethylsulfonyloxy group, n-propylsulfonyloxy group, or iso-propylsulfonyloxy group, of which a preferred example is methylsulfonyloxy group.

The term "3- to 14-membered nitrogen-containing non-aromatic heterocyclic group" used in the present specification means a monocyclic, bicyclic or tricyclic 3- to 14-membered non-aromatic heterocyclic group that may comprise one or more heteroatoms selected from the group consisting of a nitrogen atom, a sulfur atom and an oxygen atom, as well as one nitrogen atom. Preferred examples include aziridinyl group, acetidyl group, pyrrolidinyl group, pyrrolyl group, piperidinyl group, piperazinyl group, imidazolyl group, pyrazolidyl group, imidazolidyl group, morpholyl group, thiomorpholyl group, imidazolinyl group, and oxazolinyl group. Moreover, the present non-aromatic heterocyclic groups further include groups induced from a pyridone ring and non-aromatic condensed rings (e.g. groups induced from a phthalimide ring, a succinimide ring, etc.).

The substituent of the expression "may have substituent(s)" used in the present specification means one or more groups selected from the group consisting of C₁₋₈ alkyl group, C₂₋₈ alkenyl group (e.g. vinyl group), C₂₋₈ alkynyl group (e.g. ethynyl group), C₆₋₁₄ aryl group (e.g. phenyl group, etc.), 5- to 14-membered heteroaryl group (e.g. thienyl group, furyl group, pyridyl group, pyridazyl group, pyrimidyl group, pyrazyl group, etc.), hydroxyl group, C₁₋₈ alkoxy group, C₁₋₈ acyl group, C₂₋₈ acyloxy group, C₂₋₈ alkenyloxycarbonyl group, C₂₋₈ alkynyloxycarbonyl group, C₁₋₈ alkoxycarbonyl group, halogen atom, hydroxycarbonyl group, thiol group, C₁₋₈ alkylthio group, C₁₋₈ alkylsulfoxide group, C₁₋₈ alkylsulfonyl group, C₁₋₈ alkylsulfonyloxy group, hydroxysulfonyl group, nitrile group, nitro group, nitroso group, amino group, N-C₁₋₈ alkylamino group, N,N-di-C₁₋₈ alkylamino group, N-C₂₋₈ alkenylamino group, N,N-di-C₂₋₈ alkenylamino group, N-C₂₋₈ alkynylamino group, N,N-di-C₂₋₈ alkynylamino group, N-arylamino group (e.g. phenylamino group), N-heteroarylamino group (e.g. 2-pyridylamino group, 3-pyridylamino group, 1-pyrroylamino group, etc.), N-C₁₋₈ alkyl-N-arylamino group, N-C₁₋₈ alkyl-N-heteroarylamino group, aralkyloxy group, heteroaryloxy group, C₁₋₈ alkylsulfonylamino group, C₂₋₈ alkenylsulfonylamino group, C₂₋₈ alkynylsulfonylamino group, N-C₁₋₈ alkylcarbamoyl group, N-C₁₋₈ alkylcarbamoyl group, N-C₂₋₈ alkenylcarbamoyl group, N,N-di-C₂₋₈ alkynylcarbamoyl group, C₂₋₈ acylamino group, etc.

The present invention will be more specifically described in the following examples. However, these examples are not intended to limit the scope of the present invention.

In the following examples, pladienolides A, B, D and E7170 are as follows:

### Pladienolide A

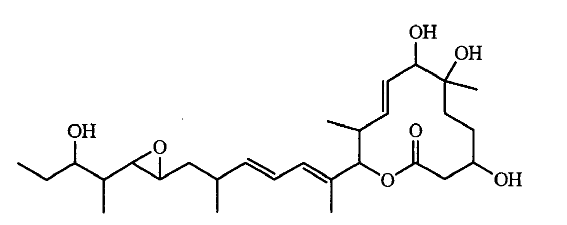

### Pladienolide B

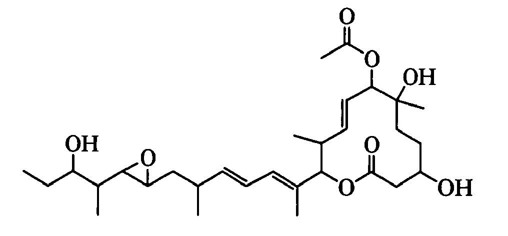

### Pladienolide C

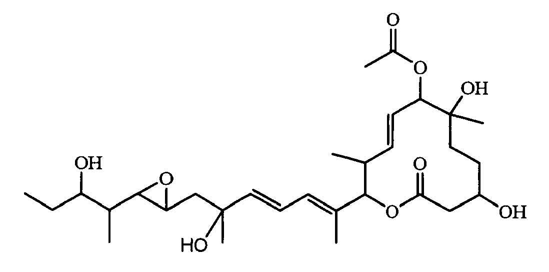

### E7107

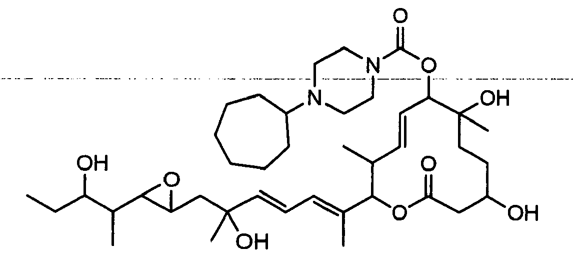

The labeled compound of the present invention was synthesized by the following method.

### Example A1

### (8E, 12E,14E)-7-(N-[G-3H]ethylcarbamoyloxy)-3.6.21-trihdroxy-6,10,12,16,20-pentamethyl-18.19-epoxytricosa-8.12.14-trien-11-olide

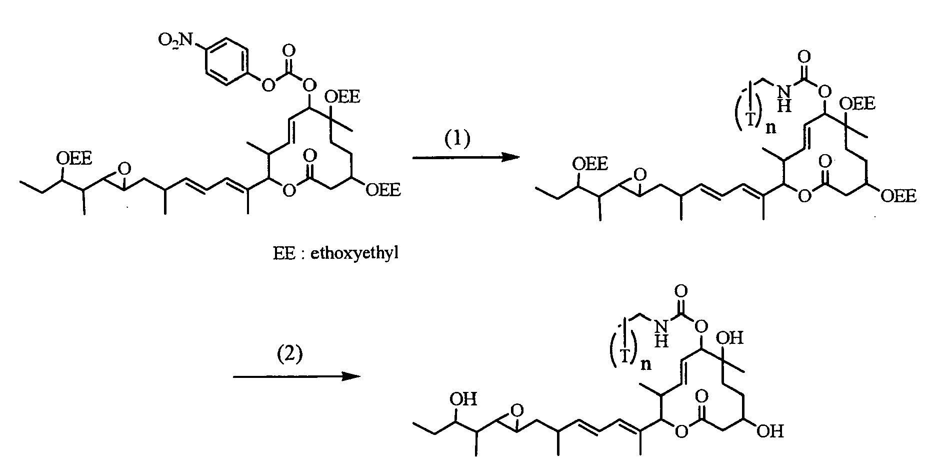

### (1) 8E,12E,14E,-7-(N-[G-³H]ethylcarbamoyloxy)-3,6,21-tri(1-ethoxyethoxy)-6,10,12,16,20-pentamethyl-18,19-epoxytricosa-8,12,14-trien-11-olide

(8E, 12E, 14E)-3,6,21-tri(1-ethoxyethoxy)-6,10,12,16,20-pentamethyl-7-(4-nitrophenoxy)carboxy-18,19-epoxytricosa-8,12,14-trien-11-olide (15 mg) described in Example B44 of the Patent Document (WO02/060890) was dissolved in anhydrous THF (1.5 ml). Thereafter, [G-³H]ethylamine (8.7 Ci) was distilled and was then added dropwise to the reaction solution. The reaction solution was stirred at room temperature for 24 hours, and the solvent was then distilled away under reduced pressure. The residue was dissolved in a small amount of ethyl acetate (yield: 680 mCi). The obtained solution was purified by silica gel HPLC (elution solvent: n-hexane : ethyl acetate = 1 : 4 to 1 : 1), so as to obtain the titled compound (yield: 113 mCi).

### (2) (8E,12E,14E)-7-(N-[G-3H]ethylcarbamoyloxy)-3,6,21-trihydroxy-6,10,12,16,20-pentamethyl-18,19-epoxytricosa-8,12,14-trien-11-olide

(8E,12E,14E)-7-(*N*-[G-³H]ethylcarbamoyloxy)-3,6,21-tri(1-ethoxyethoxy)-6,10,12,16,20-pentamethyl-18,19-epoxytricosa-8,12,14-trien-11-olide (113 mCi) and pyridinium p-toluenesulfonate (0.75 mg) were dissolved in methanol (1 ml). The reaction solution was stirred at room temperature for 4.5 hours. Thereafter, the solvent was evaporated from the reaction solution under nitrogen gas flow, and the obtained residue was then dissolved in ethyl acetate. The obtained solution was passed through a silica gel short column, so as to obtain a roughly purified product (105 mCi).

The roughly purified product was purified by silica gel HPLC (elution solvent: n-hexane : 2-propanol = 6 : 4). Thereafter, fractions of interest were combined, and the solvent was then distilled away. The residue was desiccated and was then dissolved in ethanol (105 mCi). Then, an ethanol solution that contained the product of interest of 1 mCi/ml was prepared, and it was then stored.
TOF-MS m/z 590, 592(main peak), 594(M+Na)⁺
Radiochemical purity: 97.0%(by HPLC), 97%(by TLC)
Specific activity: 56Ci/mmol(by Mass Spectroscopy)

### Example A2

### 7-(N(2-(2-(2-(N-(5-(5,5-difluoro-7,9-dimethyl-5H-dipyrrolol[2-c:2',1'-f][1,3,2]diaza borinin-4-ium-5-uid-3-yl)pentanoyl)amino)ethoxy)ethoxy)ethyl)carbamoyloxy)-3,6,21 -trihydroxy-6,10,12,16,20-pentamethyl-18,19-epoxytricosa-8,12,14-trien-11-olide

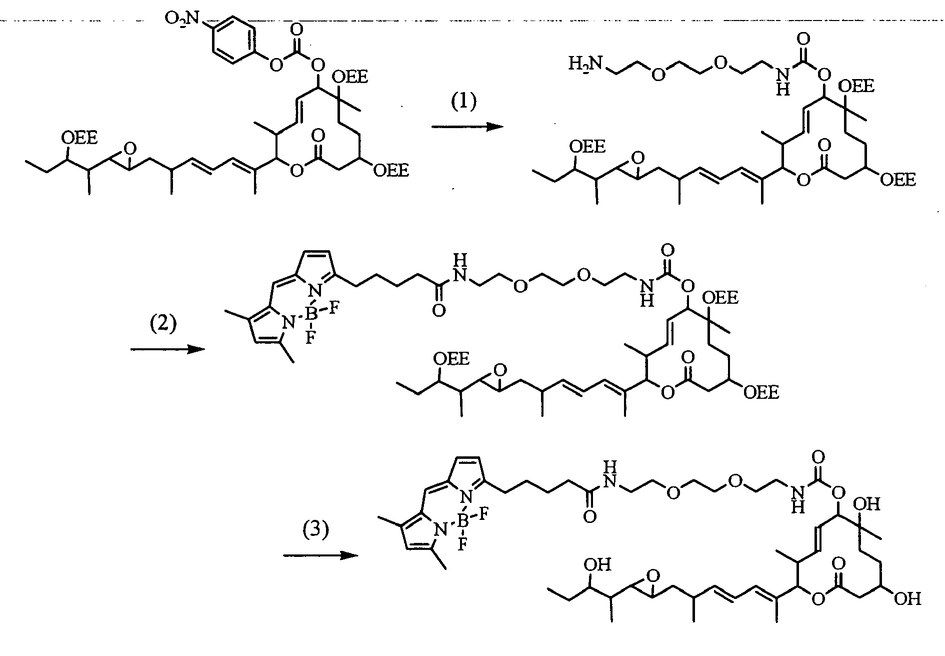

### (1) (8E,12E,14E)-7-(N-(2-(2-(2-aminoethoxy)ethoxy)ethyl)carbamoyloxy)-3,6,21-tris(1-ethoxyethoxy)-6,10,12,16,20-pentamethyl-18,19-epoxytricosa-8,12,14-trien-11-olide

1,8-diamino-3,6-dioxaoctane (127 mg, 0.85 mmol) was dissolved in THF (5 ml). Thereafter, a THF solution (15 ml) of (8E,12E,14E)-3,6,21-tri(1-ethoxyethoxy)-6,10,12,16,20-pentamethyl-7-(4-nitrophenoxy)carboxy-18,19-epoxytricosa-8,12,14-tri en-11-olide (150 mg, 0.17 mmol) described in Example B44 of the Patent Document (WO02/060890) was added dropwise to the obtained solution. The reaction solution was stirred at room temperature for 3 hours, and the solvent was then distilled away under reduced pressure. The obtained residue was purified by silica gel column chromatography (Fuji Silysia; NH silica gel; CH₂Cl₂: MeOH = 10 : 1), so as to obtain the titled compound (0.10g, 66%) in the form of a light yellow oil product.
ESI-MS m/z 885(M+H)⁺.

### (2) 7-(N-(2-(2-(2-(N-(5-(5,5-difluoro-7,9-dimethyl-5H-dipyrrolo[1,2=c:2',1'-f][1,3,2]diaz borinin-4-ium-5-uid-3-yl)pentanoyl)aminolethoxy)ethoxy)ethyl)carbamoyloxyl-3,6,21 -tri(1-ethoxyerhoxy)-6,10,12,16,20-pentamethyl-18,19-epoxytricosa-8,12,14-trien-11-o lide

(8*E*,12*E*,14*E*)-7-(*N*-(2-(2-(2-aminoethoxy)ethoxy)ethyl)carbamoyloxy)-3,6,21 -tris(1-ethoxyethoxy)-6,10,12,16,20-pentamethyl-18,19-epoxytricosa-8,12,14-trien-11-olide (10.6 mg, 12 µmol) and 4,4-difluoro-5,7-dimethyl-4-bora-3a,4a-diaza-*s-*indacene-3-pentanoic acid, succinimidyl ester (Molecular Probes; 5 mg, 12 µmol) were dissolved in THF (3 ml). The reaction solution was stirred at room temperature for 12 hours. Thereafter, the solvent was distilled away under reduced pressure, and the residue was then purified by silica gel column chromatography (Kanto Chemical, spherical, neutral, CH₂Cl₂: MeOH = 10 : 1), so as to obtain the captioned compound (13.1 mg, 92%) in the form of an orange oil product.
ESI-MS m/z 1210(M+Na)⁺.

### (3) 7-(N-(2-(2-(2-(N-(5-(5,5-difluoro-7,9-dimethyl-5H-dipyrrolo[1,2-c:2',1'-f] [1,3,2]diazaborinin-4-ium-5-uid-3-yl)pentanoyl)amino)ethoxy)ethoxy)ethyl)carbamoyl oxyl-3,6,2,1-trihydroxy-6,10,12,16,20-pentamethyl-18,19-epoxytricosa-8,12,14-trien-1 1-olide

7-(*N*-(2-(2-(2-(*N*-(5-(5,5-difluoro-7,9-dimethyl-5*H*-dipyrrolo[1,2-c:2',1'*f*][1,3 ,2]diazaborinin-4-ium-5-uid-3-yl)pentanoyl)amino)ethoxy)ethoxy)ethyl)carbamoyloxy )-3,6,21-tri(1-ethoxyethoxy)-6,10,12,16,20-pentamethyl-18,19-epoxytricosa-8,12,14-tr ien-11-olide (13 mg, 11 µmol), methanol (4 ml), and pyridinium p-toluenesulfonate (2 mg, 8 µmol) were dissolved in methanol (4 ml). The reaction solution was stirred at room temperature for 24 hours. Thereafter, the solvent was distilled away under reduced pressure, and the residue was then dissolved in ethyl acetate. The obtained solution was sequentially washed with distilled water, a sodium bicarbonate aqueous solution, and a saline solution. Thereafter, the organic layer was dried over magnesium sulfate, and the solvent was then distilled away. The obtained residue was purified by preparative TLC (Merck Art. 105628; CH₂Cl₂: MeOH = 10 : 1), so as to obtain the titled compound (9.3 mg, 87%) in the form of an orange solid.
¹H-NMR
Spectrum(CD₃OD,600MHz)δ(ppm):0.91(3H,d,J=6.8Hz),0.94(3H,d,J=7.0Hz),0.98(3H,t ,J=7.5Hz),1.12(3H,d,J=6.8Hz),1.18-1.20(4H,m),1.30-1.70(8H,m),1.72-1.84(7H,m),2.2 8-2.34(5H,m),2.46-2.64(7H,m),2.69(1H,dd,J=2.1,8.1Hz),2.76(1H,dt,Jd=2.4Hz,Jt=6.0H z),2.82(2H,br),2.98(2H,t,J=7.3Hz),3.32(1H,t,J=5.5Hz),3.38-3.42(2H,m),3.52-3.60(5H, m),3.62(4H,s),3.78-3.84(1H,m),4.94(1H,d,J=9.0Hz),5.07(1H,d,J=10.7Hz),5.59(1H,dd, J=10.0,15.2Hz),5.69(1H,dd,8.4,15.1Hz),5.74(1H,dd,J=10.0,15.1Hz),6.12(1H,d,J=11.1 Hz),6.23(1H,s),6.35(1H,dd,J=10.9,15.2Hz),6.40(1H,d,4.1Hz),7.07(1H,d,J=3.8Hz),7.45 (1H,s);ESI-MS m/z 993(M+Na)⁺.

### Example A3

### (8E,12E,14E)-3,6,21-trihydroxy-6,10,12,16,20-pentamethyl-7-(N-(2-(2-(2-(N-(5-((3aR ,4R,6aS)-2-oxohexahydro-1H-thieno[3 4-d]imidazol-4-yl)pentanoyl)amino)ethoxy) ethoxy)ethyl)carbamoyloxy)-18,19-epoxytricosa-8,12,14-trien-11-olide

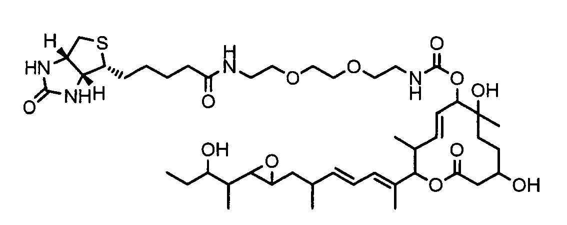

The titled compound (8 mg) was obtained in the form of a colorless oil product by the same method as that described in Example A2 with the exception that N-(+)biotinyl-1,8-diamino-3,6-dioxaoctane (Pierce; Biotin PEO-Amine) was used instead of 1,8-diamino-3,6-dioxaoctane.
¹H-NMR
Spectrum(CD₃OD,500MHz)δ(ppm):0.89(3H,d,J=5.5Hz),0.91(3H,d,J=7.0Hz),0.94(3H,t ,J=7.5Hz),1.09(3H,d,J=6.8Hz),1.16-1.26(4H,m),1.27-1.80(17H,m),2.22(2H,t,J=7.3Hz), 2.42-2.64(4H,m),2.66(1H,dd,J=2.2,8.1Hz)2.68-2.76(2H,m),2.93(1H,dd,J=4.6,12.9Hz), 2.98-3.25(1H,m),3.28-3.34(2H,m,coverd with CD3OD),3.36(2H,t,J=5.6Hz), 3.49-3.58(5H,m),3.62(4H,s),3.75-3.82(1H,m),4.31(1H,dd,J=4.4,7.8Hz),4.49(1H,dd,J= 4.9,7.8Hz),4.90(1H,d,J=9.8Hz),5.05(1H,d,J=10.7Hz),5.56(1H,dd,J=9.5,15.4Hz),5.63-5 78(2H,m),6.09(1H,d,J=10.7Hz),6.32(1H,dd,J=11.0,9,15.1Hz);ESI-MS, m/z 917(M+Na)⁺,929(M+Cl)⁻

### Example A4

### N-((1S)-5-amino-1-(methyl(2-(methyl(5-((3aR,4R,6aS)-2-oxohexahydro-1H-thieno[3,4 -d]imidazol-4-yl)pentanoyl)amino)ethyl)carbamoly)penyl)-4-(3-(trifluoromethyl)-3H diaziren-3-yl)benzamide

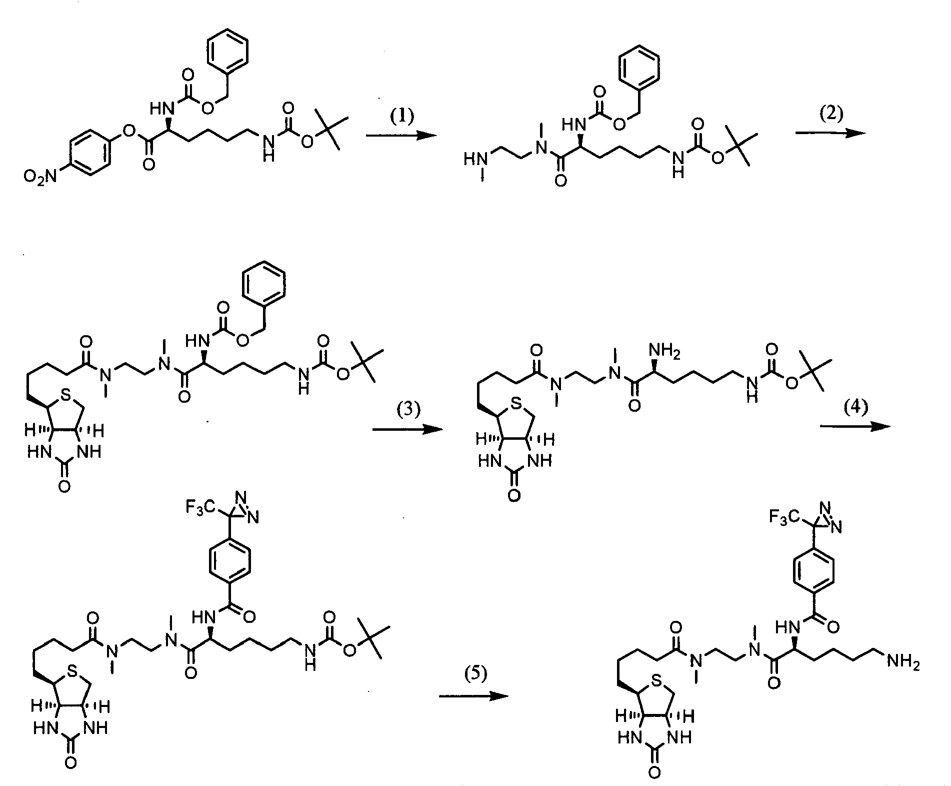

### (1) Benzyl=((1S)-5-((tert-butoxycarbonyl)amino)-1-(methyl(2-(methylamino)ethyl)carba moyl)pentyl)carbamate

1,4-dimethylethylenediamine (0.88 g, 10 mmol) was dissolved in THF (10 ml). While the obtained solution was stirred at room temperature, commercially available Z-Lys (BOC)-ONP (1 g, 2 mmol) was added thereto, dividedly several times. The reaction solution was stirred at room temperature for 12 hours. Thereafter, the solvent was distilled away, and using ethyl acetate, the residue was partitioned between oil and water. The organic layer was sequentially washed with distilled water and a saline solution, and it was then dried over magnesium sulfate. Thereafter, the solvent was distilled away. The obtained residue was purified by silica gel column chromatography (Fuji Sylysia; NH silica gel; AcOEt: MeOH = 10 : 1), so as to obtain the titled compound (900 mg, quant.) in the form of a colorless oil product.
¹H-NMR
Spectrum(CD₃Cl₃,400MHz)δ(ppm):1.30-1.78(15H,m),2.38-2.48(3H,m),2.68-2.88(2H, m),2.96(1H,s),3.00-3.16(4H,br-m),3.32-3.60(2H,m),4.58-4.76(2H,m),5.09(3H,s),5.60( 1H,d,J=8.8Hz),5.70(1H,d,J=8.0Hz),7.28-7.40(5H,m);ESI-MSm/z 473(M+Na)⁺.

### (2) Benzyl=((1S)-5-((tert-butoxycarbonyl)amino)-1-(methyl(2-(methyl(5-((3aR,4R,6aS)-2-oxohexahydro-1H-thieno[3,4-d]imidazol-4-yl)pentanoyl)amino)ethyl)carbamoyl)penty 1)carbamate

The product (0.9 g, 2 mmol) obtained in (1) above was dissolved in THF-DMF (20 ml-10 ml). While the obtained solution was stirred at room temperature, biotin N-hydroxysuccinimide (0.7 g, 2 mmol) was added thereto, dividedly several times. The reaction solution was stirred at room temperature for 12 hours. Thereafter, the solvent was distilled away, and using ethyl acetate, the residue was partitioned between oil and water. The organic layer was sequentially washed with distilled water and a saline solution, and it was then dried over magnesium sulfate. Thereafter, the solvent was distilled away. The obtained residue was purified by silica gel column chromatography (Merck Art. 1.09385; CH₂Cl₂:MeOH= 10 : 1), so as to obtain the titled compound (1.2 g, 89%) in the form of a colorless amorphous product.
¹H-NMR
Spectrum(DMSO-d₆,400MHz)δ(ppm):1.10-1.65(21H,m),2.05-3.65(17H,m),4.04-4.15( 1H,m),4.22-4.40(2H,m),4.92-5.06(2H,m),6.30-6.48(2H,m),6.70-6.80(1H,m),7.22-7.44( 6H,m);ESI-MS m/z 699(M+Na)⁺.

### (3) tert-butyl=((5S)-5-amino-6-(methyl(2-methyl(5-((3aR,4R,6aS)-2-oxohexahydro-1H-th ieno[3,4-d]imidazol-4-yl)pentanoyl-9-amino)ethyl)amino)-6-oxohexyl)carbamate

The product (1.2 g, 1.8 mmol) obtained in (2) above was dissolved in MeOH (20 ml-10 ml). A 7 M ammonia/methanol solution (20 ml) was added to the reaction solution. Thereafter, 10% palladium carbon (0.83 g) was suspended in the obtained reaction solution, followed by a catalytic reduction reaction using a hydrogenation apparatus. The yield of the reaction product was confirmed with TLC (Merck Art. 1.05719; CH₂Cl₂:MeOH = 10 : 1), and the catalyst was then removed by filtration. Thereafter, the filtrate was distilled away under reduced pressure. The obtained residue was desiccated under reduced pressure using a desiccator, so as to obtain the titled compound (0.95 g, 98%) in the form of a light yellow amorphous product.
¹H-NMR
Spectrum(DMSO-d₆,400MHz)δ(ppm):1.05-1.70(21H,m),2.10-2.40(2H,m),2.50-2.65(1 H,m),2.74-3.66(15H,m),4.06-4.20(1H,m),4.26-4.36(1H,m),6.336(1H,br-s),6.43(1H,br-s),6.70-6.84(1H,m);NH₂ was not detected as a clear peak.;ESI-MS m/z 565(M+Na)⁺.

### (4) tert-butyl=((5S)-6-methy(2-(methyl(5-((3aR,4R,6aS)-2-oxohexahydro-1H-thieno[3,4-d]imidazol-4-yl)pentanoyl)amino)ethyl)amino)-6-oxo-5-((4-(3-(trifluoromethyl)-3H-di aziren-3-yl)benzoyl)amino)hexyl)carbamate

The product (0.95 g, 1.75mmol) obtained in (3) above and 4-(3-(trifluoromethyl)-3H-diaziren-3-yl)benzoic acid (0.44 g, 1.93 mmol) synthesized in accordance with the publication (Nassal M. Liebigs Ann. Chem. 1983, 1510-1523) were dissolved in THF-DMF (20 ml-20 ml). A condensing agent, 4-(4,6-dimethoxy[1.3.5]triazin-2-yl)-4-methyl morpholium chloride hydrate (Wako; 1.05 g; 3.45 mmol), was added to the reaction solution, dividedly several times. Thereafter, the obtained reaction solution was stirred at room temperature for 24 hours. The solvent was distilled away, and using ethyl acetate, the residue was partitioned between oil and water. The organic layer was sequentially washed with distilled water and a saline solution, and it was then dried over magnesium sulfate. Thereafter, the solvent was distilled away. The obtained residue was purified by silica gel column chromatography (Merck Art. 1.09385; CH₂Cl₂: MeOH = 20 : 1 to 8 : 1), so as to obtain the captioned compound (1.18g, 89%) in the form of a colorless amorphous product.
¹H-NMR
Spectrum(DMSO-d₆,400MHz)δ(ppm):1.05-1.70(21H,m),2.10-2.3 8(2H,m),2.44-2.62(1 H,m),2.70-3.80(14H,m),4.04-4.20(1H,m),4.24-4.36(1H,m),4.70-4.90(1H, m),6.30-6.58(2H,m),6.70-6.84(1H,m),7.37(2H,d,J=8.0Hz),7.94-8.06(2H,m),8.64-8.96( 1H,m);ESI-MS m/z 777(M+Na)⁺.

### (5) N-(1S)-5-amino-1-(methyl(2-(methyl(5-((3aR,4R,6aS)-2-oxohexahydro-1H-thieno[3,4 -d]imidazol-4-yl)pentanoyl)amino)ethyl)carbamoyl)pentyl)-4-(3-(trifluoromethyl)-3H diaziren-3-yl)benzamide

The product (0.29 g, 0.38 mmol) obtained in (4) above was dissolved in ethyl acetate (20 ml), and the obtained solution was then stirred under cooling on ice. Thereafter, a 4 M hydrogen chloride-ethyl acetate solution (5 ml) was added to the obtained reaction solution, and the obtained mixture was then stirred at room temperature for 12 hours. The solvent was distilled away, and using methylene chloride and ammonia water, the residue was partitioned between oil and water. Further, the water layer was extracted again with methylene chloride. The obtained organic layers were combined. The resultant was washed with a saline solution, and it was then dried over magnesium sulfate. Thereafter, the solvent was distilled away, so as to obtain the titled compound (0.21g, 84%) in the form of a colorless amorphous product.
ESI-MS m/z 655(M+H)⁺,689(M+Cl)⁻

### Example A5

### (8E,12E,14E)-3,6,21-trihydroxy-6,10,12,16,20-pentamethyl-7-(N-((5S)-6-(N-methyl-N -(2-(N-methyl-N-(5-((3aR,4R,6aS)-2-oxohexahydro-1H-thieno[3,4-d]imidazol-4-yl)pen tanoyl)amino)ethyl)amino)-6-oxo-5-(N-(4-(3-(trifluoromethyl)-3H-diaziren-3-yl)benzo yl)amino)hexyl)carbamoyloxy)-18,19-epoxytricosa-8,12,14-trien-11-olide

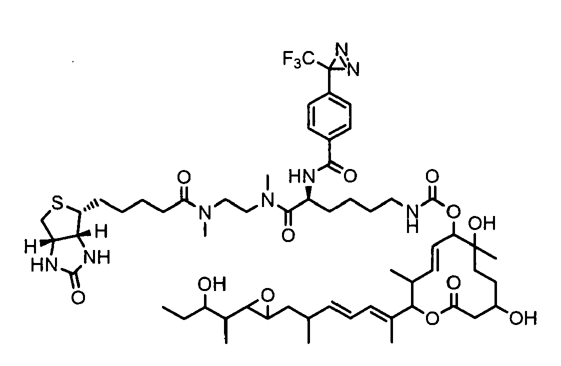

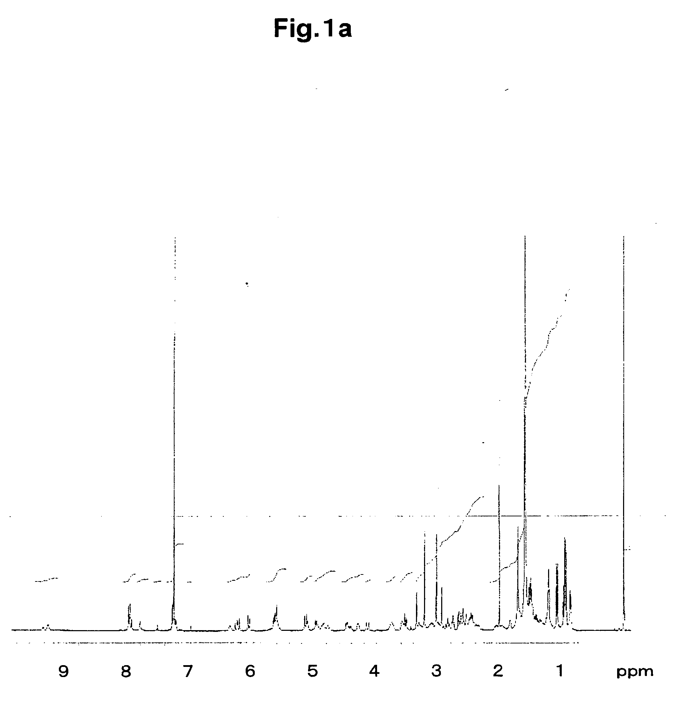

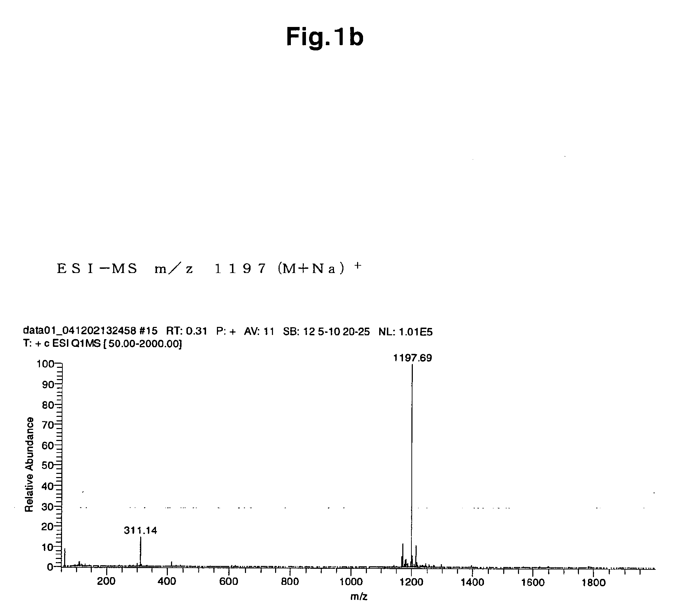

The titled compound (40 mg, 25%) was obtained in the form of a colorless oil product by the same method as that described in Example A2 using the amine compound synthesized in Example A4.
¹H-NMR Spectrum(CDCl₃,400MHz):Figure 1a, (CD₃OD, 700 MHz) δ(ppm): 0.83(3H,m),0.90(3H,d,*J*=7Hz),0.93(3H,t,*J*=7Hz),1.07(3H,d,*J*=7Hz),1.17(3H,s),1.19(1H,m),1.2 3-1.90(20H,m),1.71(3H,s),2.13-2.57(6H,m),2.62-2.74(3H,m),2.84-3.35(10H,m),3.40-4.10(6H, m),4.22-4.52(2H,m),4.80-5.10(3H,m),5.52(1H,m),5.62-5.72(2H,m),6.08(1H,d,*J*=10.4Hz),6.30
(1H,m),7.30-7.37(2H,m),7.90-8.00(2H,m)
ESI-MS m/z 1197(M+Na)⁺:Figure 1b

Using the synthesized labeled pladienolides, the target protein of pladienolides was identified as described in Examples B 1 to B7 below. In addition, it is described in Examples B2, B7, B8 and B9 that the labeled pladienolides can be used to examine that a test compound acts on (binds to) U2 snRNP, preferably SF3b, and more preferably SAP 130.

### Example B1

### Intracellular distribution of tritium probe compound

The tritiated probe compound produced in Example A1 was added to human breast cancer cells MDA-MB-468 (ATCC HTB-132) cultured on a 15-cm dish (>80% confluent), resulting in a concentration of 3 to 30 nM. The obtained mixture was then cultured in an incubator at 37°C for 1 hour. After removing the medium, the culture was then washed with a Hanks' balanced salt solution (10 ml x 2). Then, 3 ml of a 10% glycerol hypotonic buffer (10% glyrecol, 20 mM NaCl, 1 mM EDTA, 10 mM Tris-HCl, pH=7.8, protease inhibitor cocktail) was added to the resultant. The obtained mixture was left at rest for a while, and the cells were then recovered using a cell scraper. A cell lysate was prepared using a Dounce homogenizer.

The lysate was centrifuged at 2,000 g at 4°C for 10 minutes, so as to obtain a supernatant (2000 g-sup.) and a precipitate (2000 g-pellet). 2000 g-sup was further centrifuged at 100,000 g at 4°C for 60 minutes, so as to obtain a cytosol fraction as a supernatant, and a membrane fraction as a precipitate. 2000 g-pellet was suspended in 0.5 M NaCl, and the suspension was then left at rest for 30 minutes under cooling on ice, so as to elute a nuclear protein. Thereafter, centrifugation was carried out at 3,000 g at 4°C for 10 minutes, so as to obtain a nuclear fraction as a supernatant, and a nuclear pellet as a precipitate.

The radioactivity of a tritium probe existing in each fraction was measured by the following method. That is, each of the cytosol fraction and the nuclear fraction was uniformly mixed with Hionic-Fluor (Perkin Elmer). On the other hand, each of the membrane fraction and the nuclear pellet was dissolved in Soluene-350 (Packard) (1 ml), and the obtained solution was then mixed with Hionic-Fluor. The radioactivity of each mixture was measured with a liquid scintillation counter.

At the same time, a radioisotope-non-labeled-pladienolide was used to carry out a cold inhibition experiment. That is, the cells were treated with a pladienolide having a concentration (0.15 to 1.5 µM) that was 50 times higher than the tritium probe treatment concentration, and they were then cultured at 37°C in an incubator for 1 hour. Thereafter, the culture was treated with a tritium probe, and each fraction was prepared. Then, the radioactivity thereof was measured.

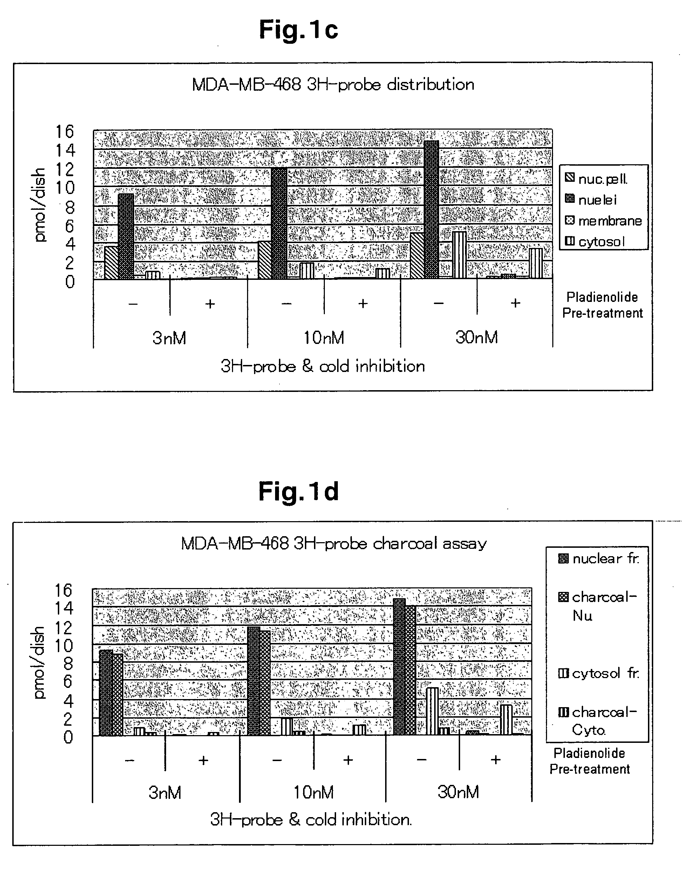

The results are shown in Figure 1c.

The highest radioactivity of the tritium probe was detected in the nuclear fraction in all the treating concentrations ranging from 3 to 30 nM. The second highest radioactivity was detected in the nuclear pellet. Such radioactivity almost completely disappeared after a pre-treatment with pladienolide B.

Moreover, a charcoal assay was carried out on the prepared cytosol fraction and nuclear fraction, so as to examine that the label count in the test sample was derived from a bound probe binding to a protein and the like, or was derived from a non-bound free form.

That is, 1/10 vol. (volume ratio) of a 10% activated charcoal suspension was added to each of the cytosol fraction and the nuclear fraction, followed by a treatment with a vortex for several seconds. The resultant was centrifuged at 15,000 rpm at 4°C for 5 minutes (TOMY mini MX-15), and the radioactivity of the supernatant was measured in the same manner as that described above.

The obtained results are shown in Figure 1d.

The radioactivity in the nuclear fraction did not disappear even after a treatment with activated charcoal In contrast, almost all the radioactivity of the cytosol fraction disappeared.

From the aforementioned results, it was suggested that a target molecule to which the pladienolide has bound exists in the nuclei.

### Example B2

### Competition assay of pladienolide analogues and derivatives with tritium probes

Various pladienolide analogues or derivatives having antitumor activity were added to human large intestine cancer WiDr cells (ATCC CCL-218) cultured on a 15-cm dish (>80% confluent), resulting in a concentration of 500 nM. The obtained mixture was cultured at 37°C in an incubator for 1 hour. Subsequently, a tritium probe compound was added to the medium, resulting in a concentration of 10 nM, and the obtained mixture was further cultured at 37°C in an incubator for 1 hour. A nuclear fraction was prepared from each sample by the same method as that described in Example B1, and the radioactivity of the fraction was then measured.

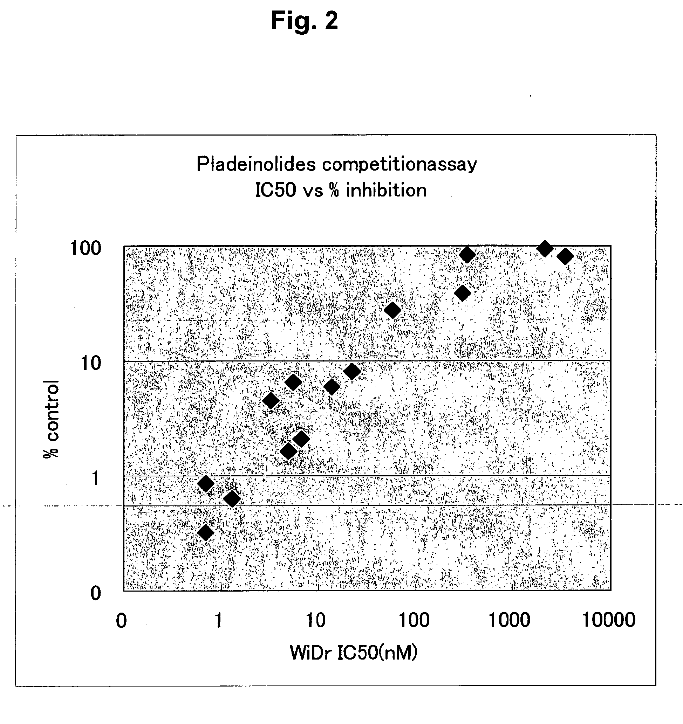

Figure 2 shows a graph in which each compound was plotted. The longitudinal axis indicates the binding rate (%) of the tritium probe compound that has bound in the presence of various pladienolide analogues or derivatives relative to control samples (no competition), and the horizontal axis indicates IC50 (nM) of the in *vitro* cytostatic activity of each compound.

The radioactivity of each sample was proportional to IC50 of the *in vitro* cytostatic activity of the competing pladienolides and the derivatives thereof to the WiDr cells. That is, stronger competitive inhibition was observed in a compound exhibiting strong cytostatic activity.

From such results, it was revealed that a target molecule that was found to exist in the nuclei as a result of examination with a tritium probe was associated with the cytostatic activity of pladienolides.
The pladienolide analogues or derivatives used in the present example are as follows:
(1) the compound of Example A3 of the present application;
(2) the compounds of Examples A-3, A-4, A-5, A-6, A-7, A-8, B-68, B-44, B-50, B36-1 and B36-2 of WO02/060890; and
(3) the compounds of Examples 12 and 45 of WO03/099813.

### Example B3

### Observation of intracellular localization of fluorescent probe

1 µM of the fluorescent probe produced in Example A2 was added to HeLa cells, and the obtained mixture was incubated at 37°C in an incubator for 1 hour. Thereafter, the medium was removed, and the remaining culture was further cultured for 1 hour in a fresh medium without probes and non-specifically adsorbed compounds were washed. The medium was removed again. The remaining culture was then washed with PBS. Thereafter, the cells were immobilized in 3.7% formaldehyde in PBS.

The immobilized cells were well washed with PBS, and were then solubilized with 0.5% or 1% Triton X-100 in PBS. Thereafter, blocking was carried out with FBS or a blocking solution, and a primary antibody (anti-SC-35 monoclonal antibody; Sigma; S4045) was added to the resultant, so as to carry out a reaction. The reaction product was washed with PBS, and blocking was carried out again with FBS or a blocking solution. A fluorescent-labeled secondary antibody (TexasRed-labeled; Jackson) was added to the resultant, so as to carry out a reaction. The labeled sample was rapidly rinsed with distilled water, and it was then encapsulated into a Prolong antifade reagent (Molecular Probes). Localization of the fluorescent probe and the anti-SC-35 antibody was observed under a fluorescence microscope (Delta Vision, Applied Precision, Inc.) or a confocal microscope (LSM510; Carl Zeiss).

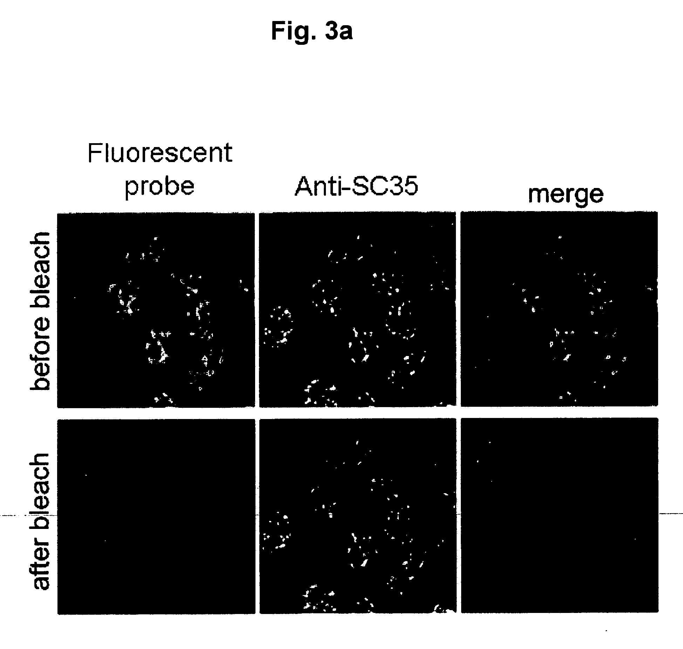

The results are shown in Figure 3a.

It was confirmed that the fluorescent probe is mainly localized in granular structures in the nuclei (Figure 3a, upper). These structures corresponded to localization of the anti-SC-35 antibody. SC-35 is a representative molecule of splicing factors and is a marker of nuclear speckles. Thus, it was suggested that the target molecule of pladienolides exists in such nuclear speckles. Moreover, the fluorescence of the fluorescent probe was faded by excessive light irradiation, and the above probe was then photographed under the same above conditions. As a result, no signals were detected in the channel of the fluorescent probe. Thus, it was confirmed that no signals were leaked from anti-SC-35 antibody staining, and that a fluorescent probe-specific signal was detected (Figure 3a, lower)

Furthermore, pladienolides or the derivatives thereof were used in the aforementioned experimental operations to carry out a competition assay. That is, before addition of the fluorescent probe, 1 µM pladienolides or the derivatives thereof were added to the cells, and the obtained mixture was then cultured for 1 hour. Thereafter, the same above operations for immobilization and staining were performed on the culture, followed by observation with a microscope, so as to confirm localization of the fluorescent probe.

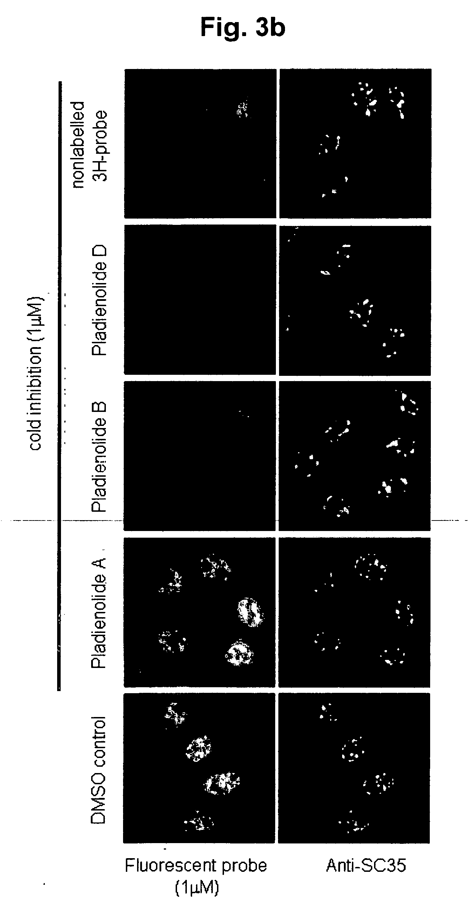

The results are shown in Figure 3b.

The fluorescence intensity derived from the fluorescent probe contained in each sample was proportional to IC50 of the cytostatic activity of the competing pladienolides or the derivatives thereof (pladienolide A >non-labeled tritium probe compound > pladienolides B and D). That is, stronger competitive inhibition was observed in a compound exhibiting strong cytostatic activity, and the fluorescence derived from the probe almost completely disappeared (Fig 3b, left column). Moreover, it was observed that the number of nuclear speckles stained with the anti-SC-35 antibody was decreased and that the size thereof was enlarged (Figure 3b, right column).

From such results, it was revealed that the target molecule confirmed to exist in the nuclear speckles by the experiments using a fluorescent probe, was associated with the cytostatic activity of pladienolides. In addition, a non-labeled tritium probe compound having a structure identical to that of a tritium probe competed with a fluorescent probe at an intensity level that was proportional to the cytostatic activity thereof. Thus, it was revealed that target molecules targeted by these two probes were identical.

### Example B4

### Immunoprecipitation experiment of nuclear fraction prepared from tritium probe-treated cells

MDA-MB-468 cells were cultured in the presence of a 30 nM tritium probe for 1 hour. Thereafter, a nuclear fraction was prepared by the same method as that described in Example B1. A dilution buffer was added to the nuclear fraction to a final concentration of 0.15 M NaCl, 0.05% CA-630 and 40 mM tris-HCl (pH=7.5), so as to prepare an immunoprecipitation solution.

To this solution, each of antibodies reacting with splicing factors, transcriptional factors, or molecules associated with such factors were added to a concentration of 1 µg/ml. The obtained mixture was slowly stirred for approximately 12 hours in a low-temperature chamber of 4°C, using a sample rotator. Thereafter, 20 µl of 50% Protein A/G-agarose conjugate suspension was added to the reaction solution, and the obtained mixture was further stirred for 2 hours in the low-temperature chamber.

The agarose resin was recovered, and it was then washed with a washing solution (0.15 M NaCl, 0.05% CA-630, 20 mM tris-HCl, pH=7.5). An SDS sample buffer was added to the resultant solution, and the obtained mixture was then stirred with a vortex. The reaction solution was boiled at 99°C for 10 minutes, so as to elute the immunoprecipitated protein. The eluant was mixed homogeneously with Hionic-Fluor, and the radioactivity of a tritium probe existing in the eluant was then measured with a liquid scintillation counter.

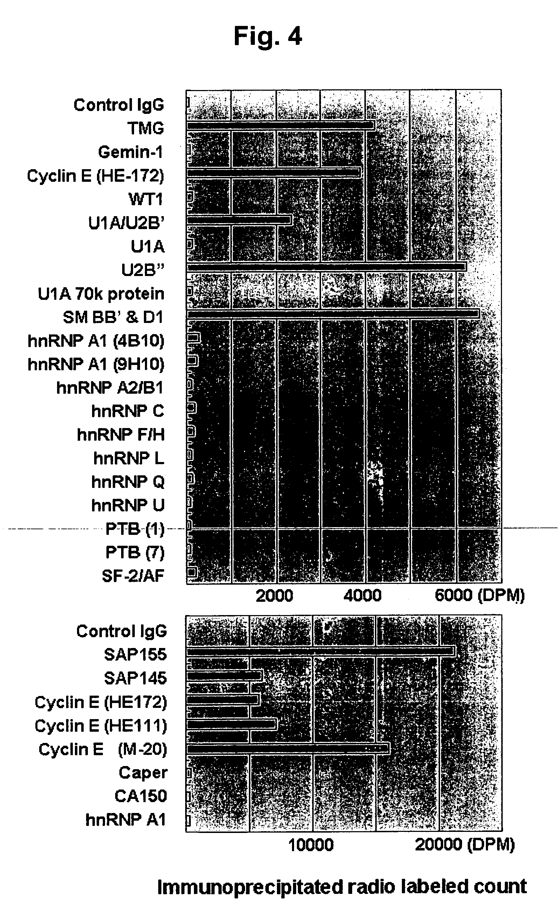

The results are shown in Figure 4.

The experiment was carried out using approximately 40 types of antibodies. As a result, specific radioactivity was observed in a TMG antibody (Oncogene NA02), a U1A/U2B" antibody (Progen 57035), a U2B"antibody (Progen 57036), an SM BB' & D1 antibody (Progen 57032), an SAP155 antibody (MBL D221-3), an SAP145 antibody (Santa Cruiz sc-14279), and a cyclin E antibody (Zymed 32-1500, Santa Cruiz sc-248, and Santa Cruiz sc-481). All of these antibodies are antibodies reacting with constitutional proteins of U2 snRNP, or a protein that reportedly forms a complex with U2 snRNP (cyclin E).

From such results, it was revealed that the target molecule of pladienolide is a protein existing in the U2 snRNP complex.

### Example B5

### Detection of bound protein by treatment with photoaffinity biotin probe

2000 g-pellet was prepared from MDA-MB-468 cells by the method described in Example B1. This pellet was suspended in a CSK buffer (300 mM sucrose, 100 mM NaCl, 3 mM MgCl₂, 10 mM PIPES, pH=7.0, protease inhibitor cocktail), and the obtained suspension was then treated with the 1.5 µM photoaffinity biotin probe synthesized in Example A3. The suspension was left at rest at 4°C for 1 hour, and it was then centrifuged at 2000 g at 4°C for 10 minutes. The supernatant was removed, and a nuclear fraction was then prepared from the obtained precipitate by the same method as that described in Example B1. Thereafter, an immunoprecipitation solution was prepared by the same method as that described in Example B4. Using antibodies regarding which immunoprecipitation of the target molecules had been proved, an immunoprecipitation experiment was carried out.

UV (365 nm and 302 nm) was applied to the obtained immunoprecipitate (agarose resin), and a crosslinking reaction was carried out. An SDS sample buffer was added to the reaction product, and the obtained mixture was then stirred with a vortex, followed by boiling at 99°C for 10 minutes, so as to elute the immunoprecipitated proteins. The eluant was subjected to SDS-PAGE and blotting onto a PVDF membrane in accordance with common methods. Thereafter, the resultant was treated with streptavidin-HRP, so as to detect probe-bound proteins.

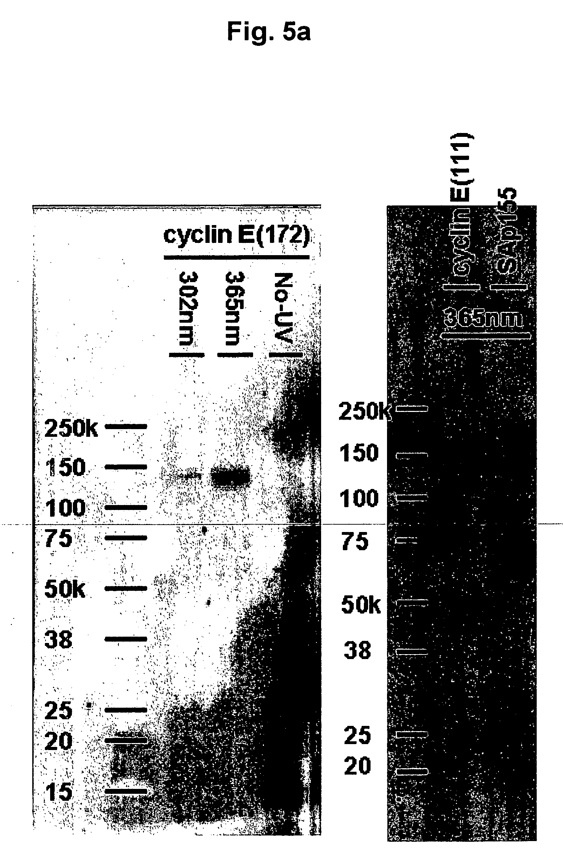

The results are shown in Figure 5a.

In all antibodies, a single band was detected at 130-140 kDa.

Subsequently, the same sample (blotting membrane) was subjected to Western blotting using an SAP155 antibody, an SAP145 antibody, and SAP 130 antibody. The band position of each of such proteins was compared with the band position of the target molecule, to which a photoaffinity probe had bound.

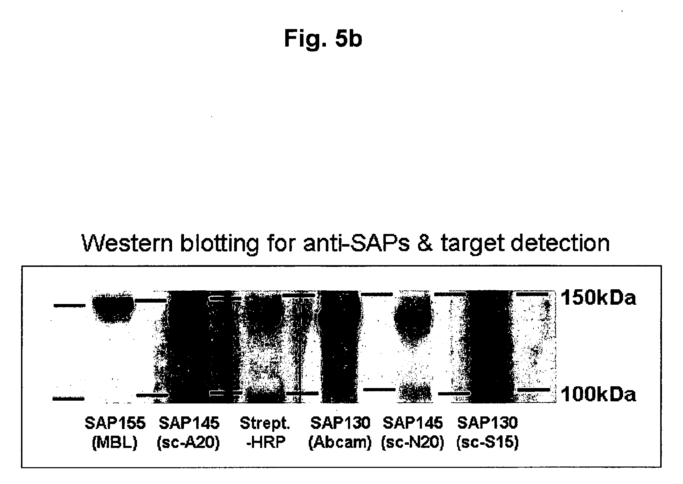

The results are shown in Figure 5b.

The position of the target molecule clearly differed from the position of SAP 155. On the other hand, the target molecule SAP145, and SAP130 were observed in almost the same position.

From such results, it was suggested that the target molecule was SAP145 or SAP130.

### Example B6

### Acquisition of GFP-145 strain and GFP-130 strain

### (1) Construction of plasmid

In order to acquire a strain stably expressing a GFP-conjugated SAP130 protein, a pEGFP-SAP130 plasmid was produced. The pEGFP-SAP130 was produced by inserting, into the NheI-PmeI site of a pcDNA3.1(-) vector (Invitrogen), an approximately 4.4 kbp DNA fragment as shown in SEQ ID NO: 1 (an NheI-PmeI fragment of GFP-SAP130; 4,432 bp), which was constituted with an EGFP protein coding sequence (derived from pEGFP-N2; CLONTECH), a human SAP130 (SF3B3) protein coding sequence (GenBank Accession#: NM_012426), and a linker sequence that connects such sequences to one another. It was anticipated that a GFP-SAP130 protein having the amino acid sequence as shown in SEQ ID NO: 2 was allowed to express in cells, into which the above plasmid had been introduced.

In order to acquire a strain stably expressing a GFP-conjugated SAP 145 protein, a pEF1-GFP-SAP145 plasmid was produced. The pEF1-GFP-SAP145 was produced by inserting, into the HindIII-PmeI site of a pEF1-Myc/His A vector (Invitrogen), an approximately 3.5 kbp DNA fragment as shown in SEQ ID NO: 3 (a HindIII-PmeI fragment of GFP-SAP145; 3,454 bp), which was constituted with an EGFP protein coding sequence (derived from pEGFP-N2; CLONTECH), a human SAP145 (SF3B2) protein coding sequence (GenBank Accession#: NM_006842), and a linker sequence that connects such sequences to one another. It was anticipated that a GFP-SAP145 protein having the amino acid sequence as shown in SEQ ID NO: 4 was allowed to express in cells, into which the above plasmid had been introduced.

### (2) Establishment of stably expressing strain

Each of the pEF1-GFP-SAP145 and pEGFP-SAP130 was introduced into HeLa cells, using Lipofectamine 2000 reagent (Inyitrogen). Forty hours after introduction of the gene, selection of gene-introduced cells was initiated by addition of Geneticin (Invitrogen) with a final concentration of 500 µg/ml. Fourteen days after the gene introduction, clones emitting GFP fluorescence were marked by fluorescence microscope observation, and were then picked up. Thereafter, a single colony was isolated by diluted passage. Clone #2 (GFP-145 strain) derived from pEF1-GFP-SAP145, and Clone #3 (GFP-130 strain) derived from pEGFP-SAP130, were used in the subsequent experiments.

### Example B7

### Detection experiment of band shift using GFP-fused SAP145 or GFP-fused SAP130 proteins

SAP145 and SAP130, which were considered to be target molecules, were detected in almost the same position on SDS-PAGE. Thus, strains stably expressing a GFP-fused SAP145 or GFP-fused SAP 130 protein were prepared, and an experiment was then carried out using a photoaffinity biotin probe.

A 1.5 µM photoaffinity biotin probe was added to HeLa cells stably expressing GFP-SAP145, and the obtained mixture was then cultured for 1 hour. After washing the cells with PBS, M-PER (PIERCE) was then added thereto, followed by stirring at 4°C for 1 hour. Thereafter, 1/10 vol. of 1.5 M NaCl-0.5% Tween 20 solution was added to the reaction solution, and the obtained mixture was then treated with a vortex (10 sec. x 3). The resultant was centrifuged at 15,000 rpm at 4°C for 5 minutes (TOMY MX-15). The thus obtained supernatant was filtrated with a 0.45-µm membrane, and the filtrate was used in the following immunoprecipitation experiment.

A GFP antibody (Q-BIOgene AFP5002) or an SAP155 antibody (MBL D221-3) (10 µg/ml) was added to the immunoprecipitation solution, and the obtained mixture was then stirred slowly with a rotator at 4°C for 30 minutes. Subsequently, 1/10 vol. of protein A/G-agarose (50% suspension) was added to the immunoprecipitation solution, and the obtained mixture was further stirred at 4°C for 1 hour in the same manner as above. The agarose resin was washed with a washing buffer 3 times, and UV was then applied to the resultant resin by the same method as that described in Example B5. Thereafter, the protein bound to the resin was eluted, and it was then subjected to SDS-PAGE and blotting. The resultant was treated with streptavidin-HRP, so as to detect a protein to which the probe had bound.

Moreover, using the same samples, SAP145 and SAP130 were detected with the SAP145 antibody and the SAP130 antibody, respectively.

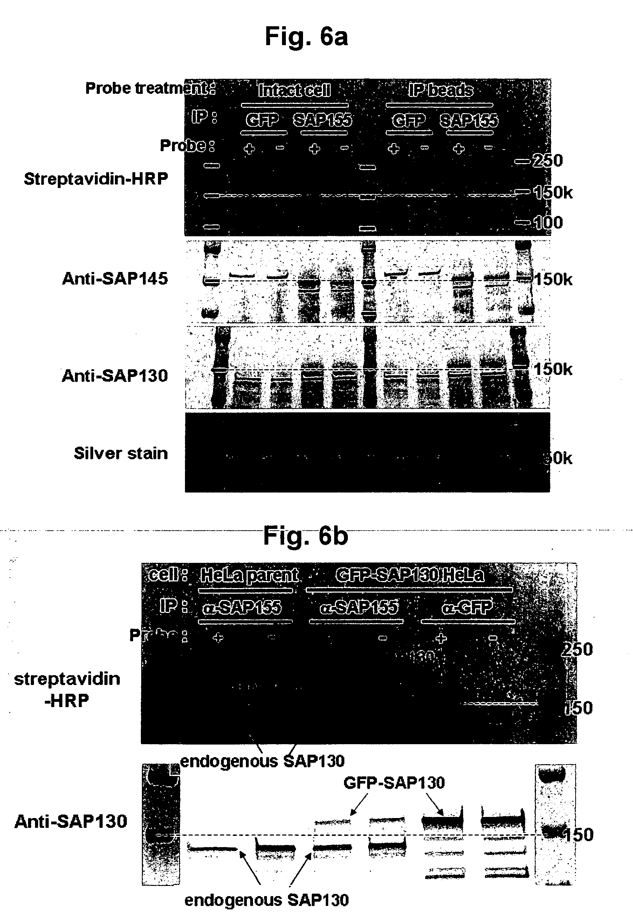

The results are shown in Figure 6a (probe treatment: intact cell).

In both samples immunoprecipitated with the GFP antibody and the SAP155 antibody, protein bands to which the probes had bound were detected in positions ranging from 130 to 145 kDa. These bands were not detected in a probe-non-treatment sample. Accordingly, it was determined that these were signals derived from the probes binding to target molecules.

The same samples were subjected to Western blotting using the SAP145 antibody and the SAP130 antibody. In the sample immunoprecipitated with the GFP antibody, no endogenous SAP145 was detected in a position ranging from 130 to 145 kDa. It was revealed that only the SAP145-GFP fusion protein was present in a shifted position of approximately 170 kDa. On the other hand, from the experimental results using the SAP130 antibody, it was revealed that SAP30 was present in a position wherein the probe had been detected.

As a result, it was revealed that SAP 145 was not present in the band position of the target molecule, and that only SAP130 was present. Thus, it was strongly suggested that the target molecule was SAP130.

Furthermore, an agarose resin prepared from probe-untreated cells was treated with a probe in the aforementioned experimental operations. Thereafter, UV irradiation and elution of a protein were carried out by the same operations as above, and it was examined whether or not the same band was detected in the obtained sample.

The results are shown in Figure 6a (probe treatment: IP beads).

Even in a case where the previously immunoprecipitated sample was treated with the probe, the same results as those in the case of the treatment of intact cells were obtained. Accordingly, it was revealed that pladienolede has affinity even for a complex comprising the immunoprecipitated target molecule.

Subsequently, the same experiment was carried out using HeLa cells stably expressing GFP-SAP130.

The results are shown in Figure 6b.

In a sample immunoprecipitated with the SAP155 antibody, two bands, namely, a band shifted to approximately 170 kDa and a band in the conventional position ranging from 130 to 145 kDa, were detected. On the other hand, in a sample immunoprecipitated with the GFP antibody, only a band shifted to 170 kDa was detected. These bands were not detected in a probe-non-treatment sample. Accordingly, it is determined that these were signals derived from the probes binding to target molecules.

The same samples were subjected to Western blotting using the SAP130 antibody. In a sample immunoprecipitated with the SAP155 antibody, signals were detected in a position wherein a band shift had been observed and in the conventional SAP130 position. On the other hand, in a sample immunoprecipitated with the GFP antibody, an SAP130 signal was observed only in the position wherein a band shift had been observed.

From such results, it was determined that a protein to which a photoaffinity biotin probe has bound is not SAP145 but SAP130.

### Example B8

### Competition assay of pladieno analogues and derivatives with photoaffinity biotin probes

Probe-untreated HeLa cells stably expressing GFP-SAP145 were subjected to an immunoprecipitation experiment by the same method as that described in Example B7 using a GFP antibody and an SAP 155 antibody, so as to prepare an immunoprecipitate sample (agarose resin). This resin was suspended in a washing buffer, and was pre-treated with pladienolides and the derivative thereof (E7107) at 75 µM, followed by stirring with a rotator at 4°C for 30 minutes. Subsequently, it was treated with a photoaffinity biotin probe at 1.5 µM, and it was further stirred for 30 minutes in the same manner as above. The resin was washed with a washing buffer. Thereafter, UV irradiation and elution of a protein were carried out by the same method as described in Example B7. The obtained sample was subjected to SDS-PAGE and blotting to examine whether or not the band derived from the probe disappeared, by the competition between the photoaffinity biotin probe and the pre-treaing pladienolide compound.

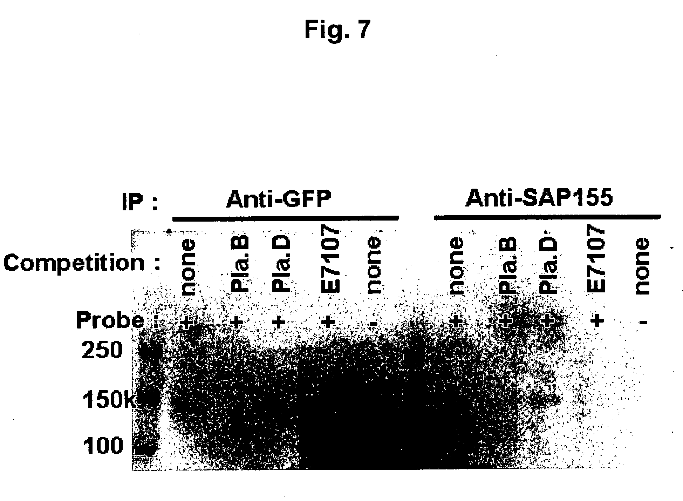

The results are shown in Figure 7.

In both cases of the GFP antibody sample and the SAP155 antibody sample, the band derived from the probe disappeared by competition with the pladienolide compounds (Pladienolide B, D and E7107). Accordingly, it can be said that the protein bounded by the photoaffinity biotin probe is the pladienolide-bound protein.

As stated above, a target molecule of pladienolide was pursued using several probe compounds. As a result, it was concluded that such a target molecule of pladienolide is SAP130, which is one of SF3b constituent proteins.

### Example B9

For the purpose of efficiently and quantitatively selecting compounds that bind to SF3b out of many evaluation samples, a simple evaluation method using High content screening machine and fluorescent probes has been established.

90 µl (1.0 x 10⁴ cells/well) of HeLa cells were inoculated on a 96-well plate used for fluorescence measurement, and they were then cultured overnight at 37°C in a 5% CO₂ incubator. A dilution series of E7107, which had been found to bind to SF3b from the results of Example B8, was prepared, and it was then added to the cultured HeLa cells to the following final concentrations (0, 0.15, 0.46, 1.4, 4.1, 12, 37, 111, 333, and 1000 nM). Thereafter, the mixture was cultured at 37°C in a 5% CO₂ incubator for 30 minutes. Subsequently, 50 µl of fluorescent probes that had been adjusted to 3 µM was added to the culture (final concentration: 1 µM), and the mixture was then reacted for 2 hours at 37°C in a 5% CO₂ incubator. The medium was removed, and the resultant was fixed with 2% paraformaldehyde for 5 minutes. The resultant was washed with PBS, was then treated with 0.1% triton X-100/PBS for 5 minutes, and was then washed with PBS. Thereafter, nuclei were stained with 1 µg/ml H33342 (SIGMA), and the image of such nuclear staining and fluorescent probes was obtained using INCell analyzer (GE Healthcare). The obtained image was subjected to image analysis software, Developer (GE Healthcare), so as to analyze the fluorescence intensity of the fluorescent probes in the nuclei and examine whether or not accumulation of the fluorescent probes into the nuclei is inhibited by E7107 binding to SF3b.

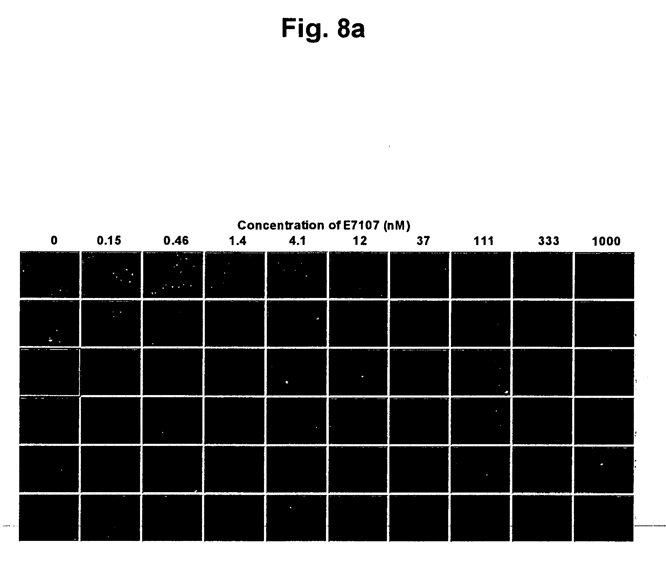

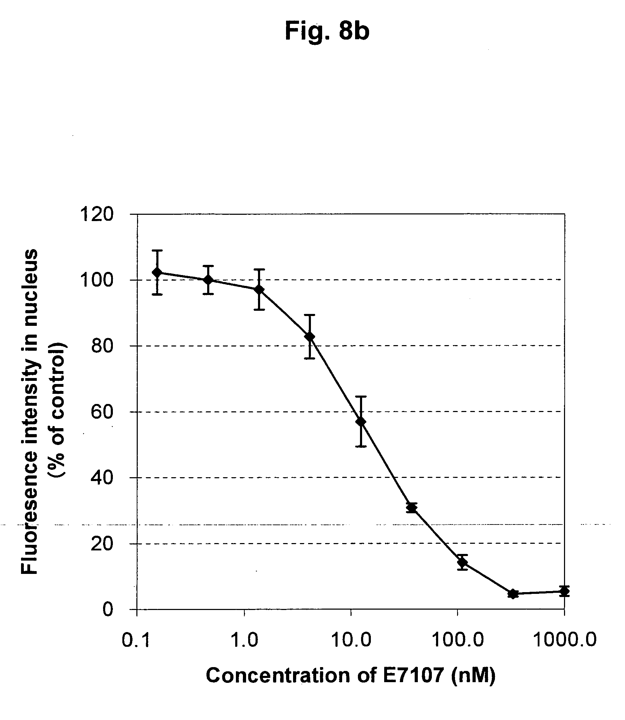

A representative example (3 views were photographed for each well) of the images of the fluorescent probes used in the image analyses (6 images in the longitudinal direction had the same concentration) is shown in Figure 8a, and the summary of such image analyses is shown in Figure 8b. E7107 concentration-dependently inhibited accumulation of the fluorescent probes into the nuclei, and thus this method enabled a simple, quantitative measurement of the compound binding to SF3b.

## Claims

1. A method of measuring the binding activity of a test compound to a splicing factor 3b (SF3b), which comprises the following steps of:
(a) contacting a labeled compound represented by the following formula (I) and a test compound with a cell or a cell fraction; and
(b) measuring the distribution of the bound labeled compound, 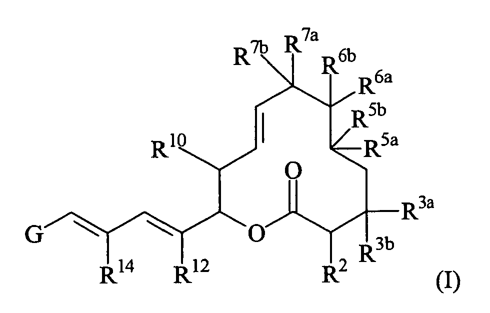 [wherein, in the above formula (I),
R², R¹⁰, R¹², and R¹⁴ are the same as or different from one another and each represents hydrogen or methyl;
R^{3a}, R^{3b}, R^{5a}, R^{5b}, R^{6a}, and R^{6b} are the same as or different from one another and each represents,
(1) hydrogen,
(2) hydroxy,
(3) any one of the following groups, each of which may have substituent(s),
(a) C₁₋₂₂ alkyl,
(b) C₁₋₂₂ alkoxy,
(c) ArCH₂O- (wherein Ar represents C₆₋₁₄ aryl or 5- to 14-membered ring heteroaryl, each of which may have substituent(s)),
(d) C₂₋₂₂ acyloxy,
(e) C₃₋₂₂ unsaturated acyloxy,
(f) -OCOR^{CO} (wherein R^{CO} represents
(i) C₆₋₁₄ aryl,
(ii) 5- to 14-membered ring heteroaryl,
(iii) C₁₋₂₂ alkoxy,
(iv) unsaturated C₂₋₂₂ alkoxy,
(v) C₆₋₁₄ aryloxy, or
(vi) 5- to 14-membered ring heteroaryloxy, each of which may have substituent(s)),
(g) C₁₋₂₂ alkylsulfonyloxy,
(h) benzenesulfonyloxy, or
(i) -OSiR^{S1}R^{S2}R^{S3} (wherein R^{s1}, R^{s2}, and R^{s3} are the same as or different from one another and each represents methyl, ethyl, i-propyl, t-butyl, or phenyl),
(4) halogen, or
(5) -R^{M}-NR^{N1}R^{N2} {wherein R^{M} represents a single bond or -O-CO-; and R^{N1} and R^{N2} are as follows,
1) R^{N1} and R^{N2} are the same as or different from each other and each represents (a) hydrogen or
(b) any one of the following groups, each of which may have substituent(s):
(i) C₁₋₂₂ alkyl,
(ii) unsaturated C₃₋₂₂ alkyl,
(iii) C₂₋₂₂ acyl,
(iv) unsaturated C₃₋₂₂ acyl,
(v) C₆₋₁₄ aryl,
(vi) 5- to 14-membered ring heteroaryl,
(vii) benzyl,
(viii) C₁₋₂₂ alkylsulfonyl or
(ix) benzenesulfonyl, or
2) NR^{N1}R^{N2} may together represent 3- to 14-membered ring nitrogen-containing nonaromatic hetero ring, which may have substituent(s)};
R^{7a} and R^{7b} are as follows,
(1) R^{7a} and R^{7b} are the same as or different from each other and each represents,
1) hydrogen,
2) -OR^{H} (wherein R^{H} represents hydrogen, methyl, or acetyl),
3) -OR^{D} {wherein R^{D} represents any one of the following groups, each of which may have substituent(s),
(i) C₁₋₂₂ alkyl (in the case of methyl, it must have substituent(s)),
(ii) -CH₂Ar,
(iii) C₃₋₂₂ acyl,
(iv) C₃₋₂₂ unsaturated acyl,
(v) -COR^{CO},
(vi) C₁₋₂₂ alkylsulfonyl,
(vii) benzenesulfonyl or
(viii) -SiR^{S1}R^{S2}R^{S3}}, or
4) -R^{M}-NR^{N1}R^{N2}, or
(2) R^{7a} and R^{7b} together represent
1) a ketone structure (=O) or
2) an oxime structure (=NOR^{OX}, wherein P^{OX} represents
(a) C₁₋₂₂ alkyl,
(b) unsaturated C₃₋₂₂ alkyl,
(c) C₆₋₁₄ aryl,
(d) 5- to 14-membered ring heteroaryl, or
(e) benzyl, each of which may have substituent(s));
further,
R^{3a} and R^{3b} may together represent a ketone structure (=O) or an oxime structure (=NOR^{OX});
still further,
R^{6a} and R^{6b} may together represent a spiro-oxirane ring or exomethylene; and still further,
either R^{6a} or R^{6b} and either R^{7a} or R^{7b} may together form a 1,3-dioxolane ring;
G represents, 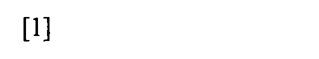 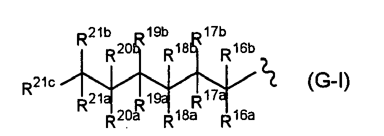 {wherein R^{16a} and R^{16b} are the same as or different from each other and each represents hydrogen, methyl, or hydroxyl;
R^{17a}, R^{17b}, R^{18a}, R^{18b}, R^{19a}, R^{19b}, R^{20a}, R^{20b}, R^{21a}, and R^{21b} are the same as or different from one another and each represents,
(1) hydrogen,
(2) methyl, which may have substituent(s),
(3) -OR^{H},
(4) -OR^{D},
(5) halogen, or
(6) -R^{M}-NR^{N1}R^{N2};
R^{21c} represents (1) hydrogen or (2) 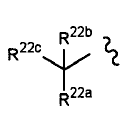 (wherein R^{22a}, R^{22b}, and R^{22c} are the same as or different from one another and each represents (a) hydrogen, (b) methyl, (c) hydroxy, (d) -OR^{H}, (e) -OR^{D}, (f) -R^{M}-NR^{N1}R^{N2}, or (g) halogen; further,
either R^{18a} or R^{18b} and either R^{19a} or R^{19b} may together form a single bond, so as to represent a partial structure 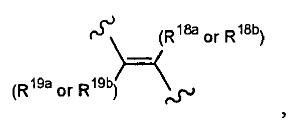 or they may bind to oxygen, so as to represent a partial structure 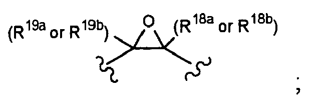 still further,
either R^{19a} or R^{19b} and either R^{20a} or R^{20b} may together form a single bond, so as to represent 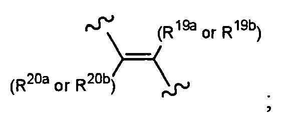 still further,
R^{21a} and R^{21b} may together represent (a) a ketone structure (=O) or (b) an oxime structure (=NOR^{OX});
still further,
either R^{21a} or R^{21b} and either R^{22a} or R^{22b} may together represent a partial structure 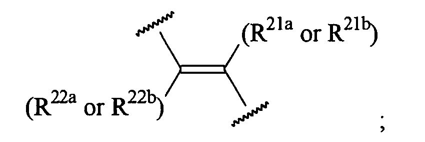 still further,
either R^{19a} or R^{19b} and either R^{21a} or R^{21b} may together represent a partial structure 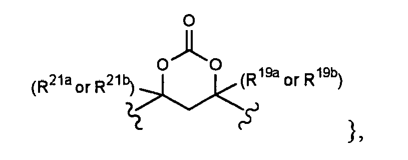 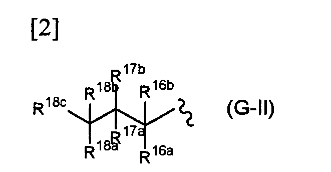 {wherein R^{16a}, R^{16b}, R^{17a}, R^{17b}, R^{18a}, and R^{18b} have the same definitions as those described in formula (G-I); and R^{18c} represents (1) hydrogen or (2) the formula: 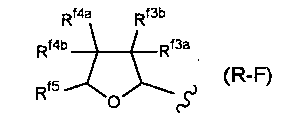 (wherein R^{f3a}, R^{f3b}, R^{f4a}, and R^{f4b} are the same as or different from one another and each represents hydrogen, methyl, hydroxy, methoxy, or acetoxy, and R^{f5} represents methyl or ethyl)} or 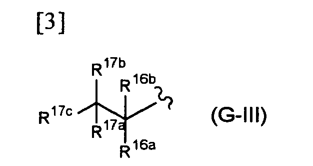 {wherein R^{16a}, R^{16b}, R^{17a}, and R^{17b} have the same definitions as those described in formula (G-I); and R^{17c} represents (1) hydrogen or (2) the formula: 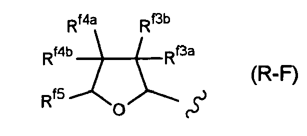 (wherein R^{f3a}, R^{f3b}, R^{f4a}, and R^{f4b} are the same as or different from one another and each represents hydrogen, methyl, hydroxy, methoxy, or acetoxy, and R^{f5} represents methyl or ethyl)}].

2. The method according to claim 1, wherein the compound represented by the above formula (I) is a compound represented by the following formula (IV): 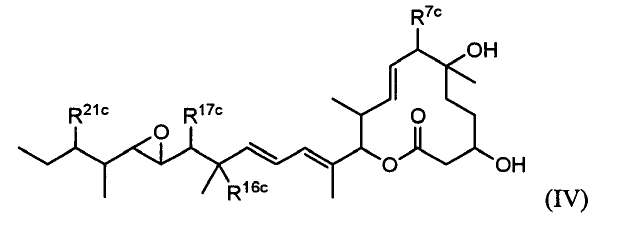 [wherein, in the above formula (IV),
R^{16c}, R^{17c}, and R^{21c} are the same as or different from one another and each represents hydrogen, hydroxy, or methoxymethyl; and
R^{7c} represents hydroxy, acetoxy, or O-CO-NR^{N1'}R^{N2'} (wherein R^{N1'} and R^{N2'} are the same as or different from one another and each represents hydrogen or C₁₋₆ alkyl)].

3. The method according to claim 1, wherein the compound represented by the above formula (I) is a compound represented by the following formula (V): 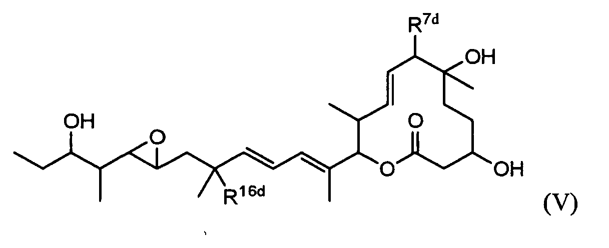 [wherein, in the above formula (V),
R^{16d} represents hydrogen or hydroxy; and
R^{7d} represents hydroxy, acetoxy, or O-CO-NHR^{N1'} (wherein R^{N1'} represents C₁₋₆ alkyl)].

4. The method according to any one of claims 1 to 3, wherein the activity of the test compound to bind to SAP130 in SF3b is measured.

5. The method according to any one of claims 1 to 4, wherein the label is radioisotope label.

6. The method according to any one of claims 1 to 4, wherein the label is fluorescent label.

7. The method according to any one of claims 1 to 4, wherein the label is biotin label.

8. The method according to any one of claims 1 to 4, wherein the label is a label with a compound that binds to a protein as a result of exposure to light.

9. The method according to any one of claims 1 to 4, wherein, in the step (b), the amount of the labeled compound that is distributed in nuclei is measured, so as to measure the binding activity of the test compound to SF3b.

10. The method according to any one of claims 1 to 4, wherein, in the step (b), the amount of the labeled compound that is distributed in nuclear speckles is measured, so as to measure the binding activity of the test compound to SF3b.

11. The method according to any one of claims 1 to 4, wherein, in the step (b), the the pattern of distribution of the labeled compound in nuclear speckles is measured, so as to measure the binding activity of the test compound to SF3b.

12. The method according to any one of claims 1 to 4, wherein, in the step (b), the amount of the labeled compound that is distributed in SF3b-bound state is measured, so as to measure the binding activity of the test compound to SF3b.

13. The method according to any one of claims 1 to 4, wherein, in the step (b), the amount of the labeled compound that is distributed in SAP130-bound state is measured, so as to measure the binding activity of the test compound to SF3b.

14. An anticancer agent binding to SF3b determined to have activity to bind to SF3b by the method according to any one of claims 1 to 13.

15. An anticancer agent binding to SAP130 determined to have activity to bind to SAP130 by the method according to any one of claims 1 to 13.

16. A labeled compound that is any one of those represented by the following formula (II):


SEQUENCE LISTING

<110> EISAI R&D MANAGEMENT CO., LTD.

<120> A target molecule of Pladienolides, a compound which interacts
with the target and a screening method thereof

<130> PCT07-0035

<150> US 60/821,199
<151> 2006-08-02

<160> 4

<170> PatentIn version 3.3

<210> 1
<211> 4432
<212> DNA
<213> Homo sapisens

<220>
<221> CDS
<222> (16)..(4416)

<400> 1
gctagcgaat tcacc atg gtg agc aag ggc gag gag ctg ttc acc ggg gtg 51
Met Val Ser Lys Gly Glu Glu Leu Phe Thr Gly Val
1               5                   10

gtg ccc atc ctg gtc gag ctg gac ggc gac gta aac ggc cac aag ttc 99
Val Pro Ile Leu Val Glu Leu Asp Gly Asp Val Asn Gly His Lys Phe
        15                  20                  25

agc gtg tcc ggc gag ggc gag ggc gat gcc acc tac ggc aag ctg acc 147
Ser Val Ser Gly Glu Gly Glu Gly Asp Ala Thr Tyr Gly Lys Leu Thr
    30                  35                  40

ctg aag ttc atc tgc acc acc ggc aag ctg ccc gtg ccc tgg ccc acc 195
Leu Lys Phe Ile Cys Thr Thr Gly Lys Leu Pro Val Pro Trp Pro Thr
45                  50                  55                  60

ctc gtg acc acc ctg acc tac ggc gtg cag tgc ttc agc cgc tac ccc 243

Leu Val Thr Thr Leu Thr Tyr Gly Val Gln Cys Phe Ser Arg Tyr Pro
                65                  70                  75

gac cac atg aag cag cac gac ttc ttc aag tcc gcc atg ccc gaa ggc 291
Asp His Met Lys Gln His Asp Phe Phe Lys Ser Ala Met Pro Glu Gly
            80                  85                  90

tac gtc cag gag cgc acc atc ttc ttc aag gac gac ggc aac tac aag 339
Tyr Val Gln Glu Arg Thr Ile Phe Phe Lys Asp Asp Gly Asn Tyr Lys
        95                  100                 105

acc cgc gcc gag gtg aag ttc gag ggc gac acc ctg gtg aac cgc atc 387
Thr Arg Ala Glu Val Lys Phe Glu Gly Asp Thr Leu Val Asn Arg Ile
    110                 115                 120

gag ctg aag ggc atc gac ttc aag gag gac ggc aac atc ctg ggg cac 435
Glu Leu Lys Gly Ile Asp Phe Lys Glu Asp Gly Asn Ile Leu Gly His
125                 130                 135                 140

aag ctg gag tac aac tac aac agc cac aac gtc tat atc atg gcc gac 483
Lys Leu Glu Tyr Asn Tyr Asn Ser His Asn Val Tyr Ile Met Ala Asp
                145                 150                 155

aag cag aag aac ggc atc aag gtg aac ttc aag atc cgc cac aac atc 531
Lys Gln Lys Asn Gly Ile Lys Val Asn Phe Lys Ile Arg His Asn Ile
            160                 165                 170

gag gac ggc agc gtg cag ctc gcc gac cac tac cag cag aac acc ccc 579
Glu Asp Gly Ser Val Gln Leu Ala Asp His Tyr Gln Gln Asn Thr Pro
        175                 180                 185

atc ggc gac ggc ccc gtg ctg ctg ccc gac aac cac tac ctg agc acc 627
Ile Gly Asp Gly Pro Val Leu Leu Pro Asp Asn His Tyr Leu Ser Thr
    190                 195                 200

cag tcc gcc ctg agc aaa gac ccc aac gag aag cgc gat cac atg gtc 675
Gln Ser Ala Leu Ser Lys Asp Pro Asn Glu Lys Arg Asp His Met Val
205                 210                 215                 220

ctg ctg gag ttc gtg acc gcc gcc ggg atc act ctc ggc atg gac gag 723
Leu Leu Glu Phe Val Thr Ala Ala Gly Ile Thr Leu Gly Met Asp Glu
                225                 230                 235

ctg tac aag ctc gag gaa ttc gtc gac ggt acc ggg ccc atg ttt ctg 771

Leu Tyr Lys Leu Glu Glu Phe Val Asp Gly Thr Gly Pro Met Phe Leu
            240                 245                 250

tac aac tta acc ttg cag aga gcc act ggc atc agc ttt gcc att cat 819
Tyr Asn Leu Thr Leu Gln Arg Ala Thr Gly Ile Ser Phe Ala Ile His
        255                 260                 265

gga aac ttt tct gga acc aaa caa caa gaa att gtt gtt tcc cgt ggg 867
Gly Asn Phe Ser Gly Thr Lys Gln Gln Glu Ile Val Val Ser Arg Gly
    270                 275                 280

aag atc ttg gag ctg ctt cgc cca gac ccc aac act ggc aaa gta cat 915
Lys Ile Leu Glu Leu Leu Arg Pro Asp Pro Asn Thr Gly Lys Val His
285                 290                 295                 300

acc cta ctc act gtg gaa gta ttc ggt gtt atc cgg tca ctc atg gcc 963
Thr Leu Leu Thr Val Glu Val Phe Gly Val Ile Arg Ser Leu Met Ala
                305                 310                 315

ttt agg ctg aca ggt ggc acc aaa gac tac att gta gtt ggc agt gac 1011
Phe Arg Leu Thr Gly Gly Thr Lys Asp Tyr Ile Val Val Gly Ser Asp
            320                 325                 330

tct ggt cga att gtt att ttg gaa tac cag cca tct aag aat atg ttt 1059
Ser Gly Arg Ile Val Ile Leu Glu Tyr Gln Pro Ser Lys Asn Met Phe
        335                 340                 345

gag aag att cac caa gaa acc ttt ggc aag agt gga tgc cgt cgc atc 1107
Glu Lys Ile His Gln Glu Thr Phe Gly Lys Ser Gly Cys Arg Arg Ile
    350                 355                 360

gtt cct ggc cag ttc tta gct gtg gat ccc aaa ggg cga gcc gtt atg 1155
Val Pro Gly Gln Phe Leu Ala Val Asp Pro Lys Gly Arg Ala Val Met
365                 370                 375                 380

att agt gcc att gag aaa cag aaa ttg gtg tat att ttg aac aga gat 1203
Ile Ser Ala Ile Glu Lys Gln Lys Leu Val Tyr Ile Leu Asn Arg Asp
                385                 390                 395

gct gca gcc cga ctt acc att tca tct ccc ctg gaa gcc cac aaa gca 1251
Ala Ala Ala Arg Leu Thr Ile Ser Ser Pro Leu Glu Ala His Lys Ala
            400                 405                 410

aac act tta gtg tat cat gta gtt gga gta gat gtc gga ttt gaa aat 1299

Asn Thr Leu Val Tyr His Val Val Gly Val Asp Val Gly Phe Glu Asn
        415                 420                 425

cca atg ttt gct tgt ctg gaa atg gat tat gag gaa gca gac aat gat 1347
Pro Met Phe Ala Cys Leu Glu Met Asp Tyr Glu Glu Ala Asp Asn Asp
    430                 435                 440

cca aca ggg gaa gca gca gct aat acc cag cag aca ctt act ttc tat 1395
Pro Thr Gly Glu Ala Ala Ala Asn Thr Gln Gln Thr Leu Thr Phe Tyr
445                 450                 455                 460

gag cta gac ctt ggt tta aat cat gtg gtc cga aaa tac agt gaa cct 1443
Glu Leu Asp Leu Gly Leu Asn His Val Val Arg Lys Tyr Ser Glu Pro
                465                 470                 475

ttg gag gaa cac ggc aac ttc ctt att aca gtt cca gga ggg tca gat 1491
Leu Glu Glu His Gly Asn Phe Leu Ile Thr Val Pro Gly Gly Ser Asp
            480                 485                 490

ggt cca agt gga gta ctg atc tgc tct gaa aac tat att act tac aag 1539
Gly Pro Ser Gly Val Leu Ile Cys Ser Glu Asn Tyr Ile Thr Tyr Lys
        495                 500                 505

aac ttt ggt gac cag cca gat atc cgc tgt cca att ccc agg agg cgg 1587
Asn Phe Gly Asp Gln Pro Asp Ile Arg Cys Pro Ile Pro Arg Arg Arg
    510                 515                 520

aat gac ctg gat gac cct gaa aga gga atg att ttt gtc tgc tct gca 1635
Asn Asp Leu Asp Asp Pro Glu Arg Gly Met Ile Phe Val Cys Ser Ala
525                 530                 535                 540

acc cat aaa acc aaa tcg atg ttc ttc ttt ttg gct caa act gag cag 1683
Thr His Lys Thr Lys Ser Met Phe Phe Phe Leu Ala Gln Thr Glu Gln
                545                 550                 555

gga gat atc ttt aag atc act ttg gag aca gat gaa gat atg gtt act 1731
Gly Asp Ile Phe Lys Ile Thr Leu Glu Thr Asp Glu Asp Met Val Thr
            560                 565                 570

gag atc cgg ctc aaa tat ttt gat act gta ccc gtt gct gct gcc atg 1779
Glu Ile Arg Leu Lys Tyr Phe Asp Thr Val Pro Val Ala Ala Ala Met
        575                 580                 585

tgt gtg ctt aaa aca ggg ttc ctt ttt gta gca tca gaa ttt gga aac 1827

Cys Val Leu Lys Thr Gly Phe Leu Phe Val Ala Ser Glu Phe Gly Asn
    590                 595                 600

cat tac tta tat caa att gca cat ctt gga gat gat gat gaa gaa cct 1875
His Tyr Leu Tyr Gln Ile Ala His Leu Gly Asp Asp Asp Glu Glu Pro
605                 610                 615                 620

gag ttt tca tca gcc atg cct ctg gaa gaa gga gac aca ttc ttt ttt 1923
Glu Phe Ser Ser Ala Met Pro Leu Glu Glu Gly Asp Thr Phe Phe Phe
                625                 630                 635

cag cca aga cca ctt aaa aac ctt gtg ctg gtt gat gag ttg gac agc 1971
Gln Pro Arg Pro Leu Lys Asn Leu Val Leu Val Asp Glu Leu Asp Ser
            640                 645                 650

ctc tct ccc att ctg ttt tgc cag ata gct gat ctg gcc aat gaa gat 2019
Leu Ser Pro Ile Leu Phe Cys Gln Ile Ala Asp Leu Ala Asn Glu Asp
        655                 660                 665

act cca cag ttg tat gtg gcc tgt ggt agg gga ccc cga tca tct ctg 2067
Thr Pro Gln Leu Tyr Val Ala Cys Gly Arg Gly Pro Arg Ser Ser Leu
    670                 675                 680

aga gtc cta aga cat gga ctt gag gtg tca gaa atg gct gtt tct gag 2115
Arg Val Leu Arg His Gly Leu Glu Val Ser Glu Met Ala Val Ser Glu
685                 690                 695                 700

cta cct ggt aac ccc aac gct gtc tgg aca gtg cgt cga cac att gaa 2163
Leu Pro Gly Asn Pro Asn Ala Val Trp Thr Val Arg Arg His Ile Glu
                705                 710                 715

gat gag ttt gat gcc tac atc att gtg tct ttc gtg aat gcc acc cta 2211
Asp Glu Phe Asp Ala Tyr Ile Ile Val Ser Phe Val Asn Ala Thr Leu
            720                 725                 730

gtg ttg tcc att gga gaa act gta gaa gaa gtg act gac tct ggg ttc 2259
Val Leu Ser Ile Gly Glu Thr Val Glu Glu Val Thr Asp Ser Gly Phe
        735                 740                 745

ctg ggg acc acc ccg acc ttg tcc tgc tcc tta tta gga gat gat gcc 2307
Leu Gly Thr Thr Pro Thr Leu Ser Cys Ser Leu Leu Gly Asp Asp Ala
    750                 755                 760

ttg gtg cag gtc tat cca gat ggc att cgg cac ata cga gca gac aag 2355

Leu Val Gln Val Tyr Pro Asp Gly Ile Arg His Ile Arg Ala Asp Lys
765                 770                 775                 780

aga gtc aat gag tgg aag acc cct gga aag aaa aca att gtg aag tgt     2403
Arg Val Asn Glu Trp Lys Thr Pro Gly Lys Lys Thr Ile Val Lys Cys
                785                 790                 795

gca gtg aac cag cga caa gtg gtg att gcc ctg aca gga gga gag ctg     2451
Ala Val Asn Gln Arg Gln Val Val Ile Ala Leu Thr Gly Gly Glu Leu
            800                 805                 810

gtc tat ttc gag atg gat cct tca gga cag ctg aat gag tac aca gaa     2499
Val Tyr Phe Glu Met Asp Pro Ser Gly Gln Leu Asn Glu Tyr Thr Glu
        815                 820                 825

cgg aag gag atg tca gca gat gtg gtg tgc atg agt ctg gcc aat gta     2547
Arg Lys Glu Met Ser Ala Asp Val Val Cys Met Ser Leu Ala Asn Val
    830                 835                 840

ccc cct gga gag cag cgg tct cgc ttc ctg gct gtg ggg ctt gtg gac     2595
Pro Pro Gly Glu Gln Arg Ser Arg Phe Leu Ala Val Gly Leu Val Asp
845                 850                 855                 860

aac act gtc aga atc atc tcc ctg gat ccc tca gac tgt ttg caa cct     2643
Asn Thr Val Arg Ile Ile Ser Leu Asp Pro Ser Asp Cys Leu Gln Pro
                865                 870                 875

cta agc atg cag gct ctc cca gcc cag cct gag tcc ttg tgt atc gtg     2691
Leu Ser Met Gln Ala Leu Pro Ala Gln Pro Glu Ser Leu Cys Ile Val
            880                 885                 890

gaa atg ggt ggg act gag aag cag gat gag ctg ggt gag agg ggc tcg     2739
Glu Met Gly Gly Thr Glu Lys Gln Asp Glu Leu Gly Glu Arg Gly Ser
        895                 900                 905

att ggc ttc cta tac ctg aat att ggg cta cag aac ggt gtg ctg ctg     2787
Ile Gly Phe Leu Tyr Leu Asn Ile Gly Leu Gln Asn Gly Val Leu Leu
    910                 915                 920

agg act gtc ttg gac cct gtc act ggg gat ttg tct gat act cgc act     2835
Arg Thr Val Leu Asp Pro Val Thr Gly Asp Leu Ser Asp Thr Arg Thr
925                 930                 935                 940

cgg tac ctg ggg tcc cgt cct gtg aag ctc ttc cga gtc cga atg caa     2883

Arg Tyr Leu Gly Ser Arg Pro Val Lys Leu Phe Arg Val Arg Met Gln
                945                 950                 955

ggc cag gag gca gta ttg gcc atg tca agc cgc tca tgg ttg agc tat 2931
Gly Gln Glu Ala Val Leu Ala Met Ser Ser Arg Ser Trp Leu Ser Tyr
            960                 965                 970

tct tac caa tct cgc ttc cat ctc acc cca ctg tct tac gag aca ctg 2979
Ser Tyr Gln Ser Arg Phe His Leu Thr Pro Leu Ser Tyr Glu Thr Leu
        975                 980                 985

gaa ttt gca tcg ggt ttt gcc tcg gaa cag tgt ccc gag ggc att gtg 3027
Glu Phe Ala Ser Gly Phe Ala Ser Glu Gln Cys Pro Glu Gly Ile Val
    990                 995                 1000

gcc atc tcc acc aac acc cta cgg att ttg gca tta gag aag ctc 3072
Ala Ile Ser Thr Asn Thr Leu Arg Ile Leu Ala Leu Glu Lys Leu
1005                1010                1015

ggt gct gtc ttc aat caa gta gcc ttc cca ctg cag tac aca ccc 3117
Gly Ala Val Phe Asn Gln Val Ala Phe Pro Leu Gln Tyr Thr Pro
1020                1025                1030

agg aaa ttt gtc atc cac cct gag agt aac aac ctt att atc att 3162
Arg Lys Phe Val Ile His Pro Glu Ser Asn Asn Leu Ile Ile Ile
1035                1040                1045

gaa acg gac cac aat gcc tac act gag gcc acg aaa gct cag aga 3207
Glu Thr Asp His Asn Ala Tyr Thr Glu Ala Thr Lys Ala Gln Arg
1050                1055                1060

aag cag cag atg gca gag gaa atg gtg gaa gca gca ggg gag gat 3252
Lys Gln Gln Met Ala Glu Glu Met Val Glu Ala Ala Gly Glu Asp
1065                1070                1075

gag cgg gag ctg gcc gca gag atg gca gca gca ttc ctc aat gaa 3297
Glu Arg Glu Leu Ala Ala Glu Met Ala Ala Ala Phe Leu Asn Glu
1080                1085                1090

aac ctc cct gaa tcc atc ttt gga gct ccc aag gct ggc aat ggg 3342
Asn Leu Pro Glu Ser Ile Phe Gly Ala Pro Lys Ala Gly Asn Gly
1095                1100                1105

cag tgg gcc tct gtg atc cga gtg atg aat ccc att caa ggg aac 3387

Gln Trp Ala Ser Val Ile Arg Val Met Asn Pro Ile Gln Gly Asn
1110                1115                1120

aca ctg gac ctt gtc cag ctg gaa cag aat gag gca gct ttt agt 3432
Thr Leu Asp Leu Val Gln Leu Glu Gln Asn Glu Ala Ala Phe Ser
1125                1130                1135

gtg gct gtg tgc agg ttt tcc aac act ggt gaa gac tgg tat gtg 3477
Val Ala Val Cys Arg Phe Ser Asn Thr Gly Glu Asp Trp Tyr Val
1140                1145                1150

ctg gtg ggt gtg gcc aag gac ctg ata cta aac ccc cga tct gtg 3522
Leu Val Gly Val Ala Lys Asp Leu Ile Leu Asn Pro Arg Ser Val
1155                1160                1165

gca ggg ggc ttc gtc tat act tac aag ctt gtg aac aat ggg gaa 3567
Ala Gly Gly Phe Val Tyr Thr Tyr Lys Leu Val Asn Asn Gly Glu
1170                1175                1180

aaa ctg gag ttt ttg cac aag act cct gtg gaa gag gtc cct gct 3612
Lys Leu Glu Phe Leu His Lys Thr Pro Val Glu Glu Val Pro Ala
1185                1190                1195

gct att gcc cca ttc cag ggg agg gtg ttg att ggt gtg ggg aag 3657
Ala Ile Ala Pro Phe Gln Gly Arg Val Leu Ile Gly Val Gly Lys
1200                1205                1210

ctg ttg cgt gtc tat gac ctg gga aag aag aag tta ctc cga aaa 3702
Leu Leu Arg Val Tyr Asp Leu Gly Lys Lys Lys Leu Leu Arg Lys
1215                1220                1225

tgt gag aat aag cat att gcc aat tat atc tct ggg atc cag act 3747
Cys Glu Asn Lys His Ile Ala Asn Tyr Ile Ser Gly Ile Gln Thr
1230                1235                1240

atc gga cat agg gta att gta tct gat gtc caa gaa agt ttc atc 3792
Ile Gly His Arg Val Ile Val Ser Asp Val Gln Glu Ser Phe Ile
1245                1250                1255

tgg gtt cgc tac aag cgt aat gaa aac cag ctt atc atc ttt gct 3837
Trp Val Arg Tyr Lys Arg Asn Glu Asn Gln Leu Ile Ile Phe Ala
1260                1265                1270

gat gat acc tac ccc cga tgg gtc act aca gcc agc ctc ctg gac 3882

Asp Asp Thr Tyr Pro Arg Trp Val Thr Thr Ala Ser Leu Leu Asp
1275                1280                1285

tat gac act gtg gct ggg gca gac aag ttt ggc aac ata tgt gtg 3927
Tyr Asp Thr Val Ala Gly Ala Asp Lys Phe Gly Asn Ile Cys Val
1290                1295                1300

gtg agg ctc cca cct aac acc aat gat gaa gta gat gag gat cct 3972
Val Arg Leu Pro Pro Asn Thr Asn Asp Glu Val Asp Glu Asp Pro
1305                1310                1315

aca gga aac aaa gcc ctg tgg gac cgt ggc ttg ctc aat ggg gcc 4017
Thr Gly Asn Lys Ala Leu Trp Asp Arg Gly Leu Leu Asn Gly Ala
1320                1325                1330

tcc cag aag gca gag gtg atc atg aac tac cat gtc ggg gag acg 4062
Ser Gln Lys Ala Glu Val Ile Met Asn Tyr His Val Gly Glu Thr
1335                1340                1345

gtg ctg tcc ttg cag aag acc acg ctg atc cct gga ggc tca gaa 4107
Val Leu Ser Leu Gln Lys Thr Thr Leu Ile Pro Gly Gly Ser Glu
1350                1355                1360

tca ctt gtc tat acc acc ttg tct gga gga att ggc atc ctt gtg 4152
Ser Leu Val Tyr Thr Thr Leu Ser Gly Gly Ile Gly Ile Leu Val
1365                1370                1375

cca ttc acg tcc cat gag gac cat gac ttc ttc cag cat gtg gaa 4197
Pro Phe Thr Ser His Glu Asp His Asp Phe Phe Gln His Val Glu
1380                1385                1390

atg cac ctg cgg tct gaa cat ccc cct ctc tgt ggg cgg gac cac 4242
Met His Leu Arg Ser Glu His Pro Pro Leu Cys Gly Arg Asp His
1395                1400                1405

ctc agc ttt cgc tcc tac tac ttc cct gtg aag aat gtg att gat 4287
Leu Ser Phe Arg Ser Tyr Tyr Phe Pro Val Lys Asn Val Ile Asp
1410                1415                1420

gga gac ctc tgt gag cag ttc aat tcc atg gaa ccc aac aaa caa 4332
Gly Asp Leu Cys Glu Gln Phe Asn Ser Met Glu Pro Asn Lys Gln
1425                1430                1435

aag aac gtc tct gaa gaa ctg gac cga acc cca ccc gaa gtg tcc 4377

Lys Asn Val Ser Glu Glu Leu Asp Arg Thr Pro Pro Glu Val Ser
1440                1445                1450

aag aaa ctc gag gat atc cgg acc cgc tac gcc ttc tga aagcttaagt 4426
Lys Lys Leu Glu Asp Ile Arg Thr Arg Tyr Ala Phe
1455                1460                1465

ttaaac 4432

<210> 2
<211> 1466
<212> PRT
<213> Homo sapisens

<400> 2

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Leu
225                 230                 235                 240

Glu Glu Phe Val Asp Gly Thr Gly Pro Met Phe Leu Tyr Asn Leu Thr
                245                 250                 255

Leu Gln Arg Ala Thr Gly Ile Ser Phe Ala Ile His Gly Asn Phe Ser
            260                 265                 270

Gly Thr Lys Gln Gln Glu Ile Val Val Ser Arg Gly Lys Ile Leu Glu
        275                 280                 285

Leu Leu Arg Pro Asp Pro Asn Thr Gly Lys Val His Thr Leu Leu Thr
    290                 295                 300

Val Glu Val Phe Gly Val Ile Arg Ser Leu Met Ala Phe Arg Leu Thr
305                 310                 315                 320

Gly Gly Thr Lys Asp Tyr Ile Val Val Gly Ser Asp Ser Gly Arg Ile
                325                 330                 335

Val Ile Leu Glu Tyr Gln Pro Ser Lys Asn Met Phe Glu Lys Ile His
            340                 345                 350

Gln Glu Thr Phe Gly Lys Ser Gly Cys Arg Arg Ile Val Pro Gly Gln
        355                 360                 365

Phe Leu Ala Val Asp Pro Lys Gly Arg Ala Val Met Ile Ser Ala Ile
    370                 375                 380

Glu Lys Gln Lys Leu Val Tyr Ile Leu Asn Arg Asp Ala Ala Ala Arg
385                 390                 395                 400

Leu Thr Ile Ser Ser Pro Leu Glu Ala His Lys Ala Asn Thr Leu Val
                405                 410                 415

Tyr His Val Val Gly Val Asp Val Gly Phe Glu Asn Pro Met Phe Ala
            420                 425                 430

Cys Leu Glu Met Asp Tyr Glu Glu Ala Asp Asn Asp Pro Thr Gly Glu
        435                 440                 445

Ala Ala Ala Asn Thr Gln Gln Thr Leu Thr Phe Tyr Glu Leu Asp Leu
    450                 455                 460

Gly Leu Asn His Val Val Arg Lys Tyr Ser Glu Pro Leu Glu Glu His
465                 470                 475                 480

Gly Asn Phe Leu Ile Thr Val Pro Gly Gly Ser Asp Gly Pro Ser Gly
                485                 490                 495

Val Leu Ile Cys Ser Glu Asn Tyr Ile Thr Tyr Lys Asn Phe Gly Asp
            500                 505                 510

Gln Pro Asp Ile Arg Cys Pro Ile Pro Arg Arg Arg Asn Asp Leu Asp
        515                 520                 525

Asp Pro Glu Arg Gly Met Ile Phe Val Cys Ser Ala Thr His Lys Thr
    530                 535                 540

Lys Ser Met Phe Phe Phe Leu Ala Gln Thr Glu Gln Gly Asp Ile Phe
545                 550                 555                 560

Lys Ile Thr Leu Glu Thr Asp Glu Asp Met Val Thr Glu Ile Arg Leu
                565                 570                 575

Lys Tyr Phe Asp Thr Val Pro Val Ala Ala Ala Met Cys Val Leu Lys
            580                 585                 590

Thr Gly Phe Leu Phe Val Ala Ser Glu Phe Gly Asn His Tyr Leu Tyr
        595                 600                 605

Gln Ile Ala His Leu Gly Asp Asp Asp Glu Glu Pro Glu Phe Ser Ser
    610                 615                 620

Ala Met Pro Leu Glu Glu Gly Asp Thr Phe Phe Phe Gln Pro Arg Pro
625                 630                 635                 640

Leu Lys Asn Leu Val Leu Val Asp Glu Leu Asp Ser Leu Ser Pro Ile
                645                 650                 655

Leu Phe Cys Gln Ile Ala Asp Leu Ala Asn Glu Asp Thr Pro Gln Leu
            660                 665                 670

Tyr Val Ala Cys Gly Arg Gly Pro Arg Ser Ser Leu Arg Val Leu Arg
        675                 680                 685

His Gly Leu Glu Val Ser Glu Met Ala Val Ser Glu Leu Pro Gly Asn
    690                 695                 700

Pro Asn Ala Val Trp Thr Val Arg Arg His Ile Glu Asp Glu Phe Asp
705                 710                 715                 720

Ala Tyr Ile Ile Val Ser Phe Val Asn Ala Thr Leu Val Leu Ser Ile
                725                 730                 735

Gly Glu Thr Val Glu Glu Val Thr Asp Ser Gly Phe Leu Gly Thr Thr
            740                 745                 750

Pro Thr Leu Ser Cys Ser Leu Leu Gly Asp Asp Ala Leu Val Gln Val
        755                 760                 765

Tyr Pro Asp Gly Ile Arg His Ile Arg Ala Asp Lys Arg Val Asn Glu
    770                 775                 780

Trp Lys Thr Pro Gly Lys Lys Thr Ile Val Lys Cys Ala Val Asn Gln
785                 790                 795                 800

Arg Gln Val Val Ile Ala Leu Thr Gly Gly Glu Leu Val Tyr Phe Glu
                805                 810                 815

Met Asp Pro Ser Gly Gln Leu Asn Glu Tyr Thr Glu Arg Lys Glu Met
            820                 825                 830

Ser Ala Asp Val Val Cys Met Ser Leu Ala Asn Val Pro Pro Gly Glu
        835                 840                 845

Gln Arg Ser Arg Phe Leu Ala Val Gly Leu Val Asp Asn Thr Val Arg
    850                 855                 860

Ile Ile Ser Leu Asp Pro Ser Asp Cys Leu Gln Pro Leu Ser Met Gln
865                 870                 875                 880

Ala Leu Pro Ala Gln Pro Glu Ser Leu Cys Ile Val Glu Met Gly Gly
                885                 890                 895

Thr Glu Lys Gln Asp Glu Leu Gly Glu Arg Gly Ser Ile Gly Phe Leu
            900                 905                 910

Tyr Leu Asn Ile Gly Leu Gln Asn Gly Val Leu Leu Arg Thr Val Leu
        915                 920                 925

Asp Pro Val Thr Gly Asp Leu Ser Asp Thr Arg Thr Arg Tyr Leu Gly
    930                 935                 940

Ser Arg Pro Val Lys Leu Phe Arg Val Arg Met Gln Gly Gln Glu Ala
945                 950                 955                 960

Val Leu Ala Met Ser Ser Arg Ser Trp Leu Ser Tyr Ser Tyr Gln Ser
                965                 970                 975

Arg Phe His Leu Thr Pro Leu Ser Tyr Glu Thr Leu Glu Phe Ala Ser
            980                 985                 990

Gly Phe Ala Ser Glu Gln Cys Pro Glu Gly Ile Val Ala Ile Ser Thr
        995                 1000                1005

Asn Thr Leu Arg Ile Leu Ala Leu Glu Lys Leu Gly Ala Val Phe
    1010                1015                1020

Asn Gln Val Ala Phe Pro Leu Gln Tyr Thr Pro Arg Lys Phe Val
    1025                1030                1035

Ile His Pro Glu Ser Asn Asn Leu Ile Ile Ile Glu Thr Asp His
    1040                1045                1050

Asn Ala Tyr Thr Glu Ala Thr Lys Ala Gln Arg Lys Gln Gln Met
    1055                1060                1065

Ala Glu Glu Met Val Glu Ala Ala Gly Glu Asp Glu Arg Glu Leu
    1070                1075                1080

Ala Ala Glu Met Ala Ala Ala Phe Leu Asn Glu Asn Leu Pro Glu
    1085                1090                1095

Ser Ile Phe Gly Ala Pro Lys Ala Gly Asn Gly Gln Trp Ala Ser
    1100                1105                1110

Val Ile Arg Val Met Asn Pro Ile Gln Gly Asn Thr Leu Asp Leu
    1115                1120                1125

Val Gln Leu Glu Gln Asn Glu Ala Ala Phe Ser Val Ala Val Cys
    1130                1135                1140

Arg Phe Ser Asn Thr Gly Glu Asp Trp Tyr Val Leu Val Gly Val
    1145                1150                1155

Ala Lys Asp Leu Ile Leu Asn Pro Arg Ser Val Ala Gly Gly Phe
    1160                1165                1170

Val Tyr Thr Tyr Lys Leu Val Asn Asn Gly Glu Lys Leu Glu Phe
    1175                1180                1185

Leu His Lys Thr Pro Val Glu Glu Val Pro Ala Ala Ile Ala Pro
    1190                1195                1200

Phe Gln Gly Arg Val Leu Ile Gly Val Gly Lys Leu Leu Arg Val
    1205                1210                1215

Tyr Asp Leu Gly Lys Lys Lys Leu Leu Arg Lys Cys Glu Asn Lys
    1220                1225                1230

His Ile Ala Asn Tyr Ile Ser Gly Ile Gln Thr Ile Gly His Arg
    1235                1240                1245

Val Ile Val Ser Asp Val Gln Glu Ser Phe Ile Trp Val Arg Tyr
    1250                1255                1260

Lys Arg Asn Glu Asn Gln Leu Ile Ile Phe Ala Asp Asp Thr Tyr
    1265                1270                1275

Pro Arg Trp Val Thr Thr Ala Ser Leu Leu Asp Tyr Asp Thr Val
    1280                1285                1290

Ala Gly Ala Asp Lys Phe Gly Asn Ile Cys Val Val Arg Leu Pro
    1295                1300                1305

Pro Asn Thr Asn Asp Glu Val Asp Glu Asp Pro Thr Gly Asn Lys
    1310                1315                1320

Ala Leu Trp Asp Arg Gly Leu Leu Asn Gly Ala Ser Gln Lys Ala
    1325                1330                1335

Glu Val Ile Met Asn Tyr His Val Gly Glu Thr Val Leu Ser Leu
    1340                1345                1350

Gln Lys Thr Thr Leu Ile Pro Gly Gly Ser Glu Ser Leu Val Tyr
    1355                1360                1365

Thr Thr Leu Ser Gly Gly Ile Gly Ile Leu Val Pro Phe Thr Ser
    1370                1375                1380

His Glu Asp His Asp Phe Phe Gln His Val Glu Met His Leu Arg
    1385                1390                1395

Ser Glu His Pro Pro Leu Cys Gly Arg Asp His Leu Ser Phe Arg
    1400                1405                1410

Ser Tyr Tyr Phe Pro Val Lys Asn Val Ile Asp Gly Asp Leu Cys
    1415                1420                1425

Glu Gln Phe Asn Ser Met Glu Pro Asn Lys Gln Lys Asn Val Ser
    1430                1435                1440

Glu Glu Leu Asp Arg Thr Pro Pro Glu Val Ser Lys Lys Leu Glu
    1445                1450                1455

Asp Ile Arg Thr Arg Tyr Ala Phe
    1460                1465

<210> 3
<211> 3454
<212> DNA

<213>  Homo sapisens

<220>
<221>  CDS
<222>  (28)..(3438)

<400>  3
aagcttggta cggctagcga attcacc atg gtg agc aag ggc gag gag ctg ttc      54
acc ggg gtg gtg ccc atc ctg gtc gag ctg gac ggc gac gta aac ggc      102
cac aag ttc agc gtg tcc ggc gag ggc gag ggc gat gcc acc tac ggc      150
aag ctg acc ctg aag ttc atc tgc acc acc ggc aag ctg ccc gtg ccc      198
tgg ccc acc ctc gtg acc acc ctg acc tac ggc gtg cag tgc ttc agc      246
cgc tac ccc gac cac atg aag cag cac gac ttc ttc aag tcc gcc atg      294
ccc gaa ggc tac gtc cag gag cgc acc atc ttc ttc aag gac gac ggc      342
aac tac aag acc cgc gcc gag gtg aag ttc gag ggc gac acc ctg gtg      390
aac cgc atc gag ctg aag ggc atc gac ttc aag gag gac ggc aac atc      438
ctg ggg cac aag ctg gag tac aac tac aac agc cac aac gtc tat atc 486
atg gcc gac aag cag aag aac ggc atc aag gtg aac ttc aag atc cgc 534
cac aac atc gag gac ggc agc gtg cag ctc gcc gac cac tac cag cag 582
aac acc ccc atc ggc gac ggc ccc gtg ctg ctg ccc gac aac cac tac 630
ctg agc acc cag tcc gcc ctg agc aaa gac ccc aac gag aag cgc gat 678
cac atg gtc ctg ctg gag ttc gtg acc gcc gcc ggg atc act ctc ggc 726
atg gac gag ctg tac aag ctc gac atg gcg acg gag cat ccc gag cct 774
ccc aaa gca gaa ttg cag ctg ccg ccg ccg cca cct cca ggc cac tat 822
ggc gcc tgg gct gcc cag gag ctt cag gcc aag ttg gca gag atc gga 870
gct ccg atc cag ggt aat cgc gag gag ctg gtg gag cgg ctg cag agc 918
tac acc cgc cag act ggc atc gtg ctg aat cgg ccg gtt ttg aga ggg 966
gaa gat ggg gac aaa gcc gct cca cct ccc atg tcg gca cag ctc cct 1014
gga att ccc atg cca cca cca cct ttg gga ctc ccc cct ctg cag cct 1062
cct ccg cca ccc cca cca cct cca cca ggc ctt ggc ctt ggc ttt cct 1110
atg gcc cac cca cca aat ttg ggg ccc ccg cct cct ctc cgt gtg ggt 1158
gag cca gtg gca ctg tca gag gag gag cgg ctg aag ttg gct cag cag 1206
cag gcg gca ttg ctg atg cag cag gag gag cgt gcc aag cag cag gga 1254
gat cat tcg ctg aag gaa cat gag ctc ttg gag cag cag aag cgg gca 1302
gct gtg tta ctg gag cag gaa cga cag cag gag att gcc aag atg ggc 1350
acc cca gtc cct cgg ccc cca caa gac atg ggc cag att ggt gtg cgc 1398
act cct ctg ggt cct cga gta gct gct cca gtg ggc cca gtg ggc ccc 1446
act cct aca gtt ttg ccc atg gga gcc cct gtt ccc cgg cct cgt ggt 1494
ccc cca ccg ccc cct gga gat gag aac aga gag atg gat gac ccc tct 1542
gtg ggc ccc aag atc ccc cag gct ttg gag aag atc ctg cag ctg aag 1590
gag agc cgc cag gaa gag atg aat tct cag cag gag gaa gag gaa atg 1638
gaa aca gat gct cgc tcg tcc ctg ggc cag tca gcg tca gag act gag 1686
gag gac aca gtg tcc gta tct aaa aag gag aaa aac cgg aag cgt agg 1734
aac cga aag aag aag aaa aag ccc cag cgg gtg cga ggg gtg tcc tct 1782
gag agc tct ggg gac cgg gag aaa gac tca acc cgg tcc cgt ggc tct 1830
gat tcc cca gca gct gat gtt gag att gag tat gtg act gaa gaa cct 1878
gaa att tac gag ccc aac ttt atc ttc ttt aag agg atc ttt gag gct 1926
ttt aag ctc act gat gat gtg aag aag gag aaa gag aag gag cca gag 1974
aaa ctt gac aaa ctg gag aac tct gca gcc ccc aag aag aag gga ttt 2022
gaa gag gag cac aag gac agt gat gat gac agc agt gat gac gag cag 2070
gaa aag aag cca gaa gcc ccc aag ctg tcc aag aag aag ttg cgc cga 2118
atg aac cgc ttc act gtg gct gaa ctc aag cag ctg gtg gct cgg ccc 2166
gat gtc gtg gag atg cac gat gtg aca gcg cag gac cct aag ctc ttg 2214
gtt cac ctc aag gcc act cgg aac tct gtg cct gtg cca cgc cac tgg 2262
tgt ttt aag cgc aaa tac ctg cag ggc aaa cgg ggc att gag aag ccc 2310
ccc ttc gag ctg cca gac ttc atc aaa cgc aca ggc atc cag gag atg 2358
cga gag gcc ctg cag gag aag gaa gaa cag aag acc atg aag tca aaa 2406
atg cga gag aaa gtt cgg cct aag atg ggc aaa att gac atc gac tac 2454
cag aaa ctg cat gat gcc ttc ttc aag tgg cag acc aag cca aag ctg 2502
acc atc cat ggg gac ctg tac tat gag ggg aag gag ttc gag aca cga 2550
ctg aag gag aag aag cca gga gat ctg tct gat gag cta agg att tcc 2598
ttg ggg atg cca gta gga cca aat gcc cac aag gtc cct ccc cca tgg 2646
ctg att gcc atg cag cga tat gga cca ccc cca tcg tat ccc aac ctg 2694
aaa atc cct ggg ctg aac tcg ccc atc cct gag agc tgt tcc ttt ggg 2742
tac cat gct ggt ggc tgg ggc aaa cct cca gtg gat gag act ggg aaa 2790
ccg ctc tat ggg gac gtg ttt gga acc aat gct gct gaa ttt cag acc 2838
aag act gag gaa gaa gag att gat cgg acc cct tgg ggg gaa ctg gaa 2886
cca tct gat gaa gaa tcc tca gaa gaa gag gaa gag gaa gaa agt gat 2934
gaa gac aaa cca gat gag aca ggc ttt att acc cct gca gac agt ggc 2982
ctt atc act cct gga ggc ttt tca tca gtg cct gct gga atg gag acc 3030
cct gaa ctc att gag ctg agg aag aag aag att gag gag gcg atg 3075
gac gga agt gag aca cct cag ctc ttc act gtg ttg cca gag aag 3120
aga aca gcc act gtt gga ggg gcc atg atg gga tca acc cac att 3165
tat gac atg tcc acg gtt atg agc cgg aag ggc ccg gct cct gag 3210
ctg caa ggt gtg gaa gtg gcg ctg gcg cct gaa gag ttg gag ctg 3255
gat cct atg gcc atg acc cag aag tat gag gag cat gtg cgg gag 3300
cag cag gct caa gta gag aag gag gac ttc agt gac atg gtg gct 3345
gag cac gct gcc aaa cag aag caa aaa aaa cgg aaa gct cag ccc 3390
cag gac agc cgt ggg ggc agc aag aaa tat aag gag ttc aag ttt 3435
tag aagcttaagt ttaaac 3454

<210> 4
<211> 1136
<212> PRT
<213> Homo sapisens

<400> 4